(19) Europäisches Patentamt
European Patent Office
Office européen des brevets

(11) Veröffentlichungsnummer : **0 350 002 B1**

(12) # EUROPÄISCHE PATENTSCHRIFT

(45) Veröffentlichungstag der Patentschrift : **13.01.93 Patentblatt 93/02**

(21) Anmeldenummer : **89112263.2**

(22) Anmeldetag : **05.07.89**

(51) Int. Cl.$^5$ : **C07F 9/38,** A61K 31/66, C07F 9/60, C07F 9/572, C07F 9/6506, C07F 9/59, C07F 9/6509, C07F 9/6544, C07F 9/6533

(54) **Neue Diphosphonsäurederivate, Verfahren zu deren Herstellung und diese Verbindungen enthaltende Arzneimittel.**

(30) Priorität : **05.07.88 DE 3822650**

(43) Veröffentlichungstag der Anmeldung : **10.01.90 Patentblatt 90/02**

(45) Bekanntmachung des Hinweises auf die Patenterteilung : **13.01.93 Patentblatt 93/02**

(84) Benannte Vertragsstaaten : **AT BE CH DE ES FR GB GR IT LI LU NL SE**

(56) Entgegenhaltungen :
**EP-A- 0 186 405**
**ZA-A- 879 454**

(73) Patentinhaber : **BOEHRINGER MANNHEIM GMBH**
**Sandhofer Strasse 116**
**W-6800 Mannheim 31 (DE)**

(72) Erfinder : **Bosies, Elmar, Dr. phil.nat.**
**Delpstrasse 11**
**W-6940 Weinheim (DE)**
Erfinder : **Zilch, Harald, Dr. rer.nat.**
**Alsenweg 2 A**
**W-6800 Mannheim 31 (DE)**

EP 0 350 002 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Diphosphonsäurederivate, Verfahren zu deren Herstellung sowie Arzneimittel, die diese Substanzen enthalten.

In der EP-A-0 274 158 sind 1-Hydroxy-piperidinyl-alkyliden-1,1-diphosphonate beschrieben, bei denen die Alkylkette durch ein Sauerstoffatom unterbrochen ist. Analoge Pyridyl - Verbindungen sind in EP-A-0 186 405 beschrieben.

In der DE-PS 18 13 659 sind Diphosphonsäurederivate beschrieben, von denen die 1-Hydroxyethan-1,1-diphosphonsäure als Mittel zur Behandlung von Morbus Paget Bedeutung erlangt hat.

In der DE-PS 25 34 391 sind Aminoalkan-1,1-diphosphonsäuren, die am Stickstoffatom mit $C_1$-$C_3$-Alkylgruppen substituiert sein können, mit einer Wirkung auf den Calciumstoffwechsel beschrieben. Analogu Verbindungen sind in US 4,407,761 beschrieben.

Überraschenderweise wurde nun gefunden, daß Aminoalkan-1,1-diphosphonsäuren, bei denen die Alkylkette durch ein Sauerstoffatom unterbrochen ist, eine deutlich ausgeprägtere Wirkung auf den Calciumstoffwechsel zeigen als die bisher bekannten Verbindungen. Diese Substanzen sind somit besonders zu einer breiten Behandlung von Calciumstoffwechselstörungen geeignet. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a.. Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

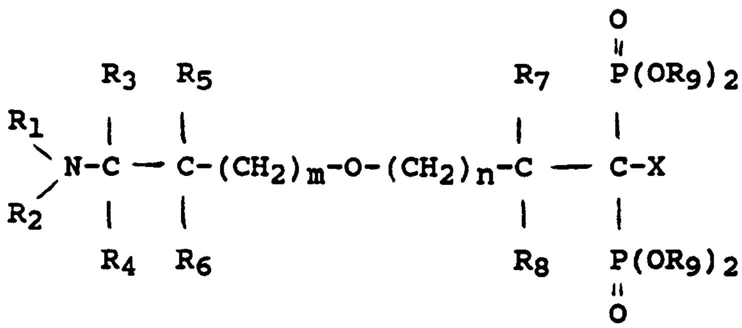

(I),

in der

$R_1$ und $R_2$ jeweils unabhängig voneinander Wasserstoff, eine geradkettige oder verzweigte, gesättigte oder ungesättigte Alkylkette mit 1-9 Kohlenstoffatomen, die gegebenenfalls durch Hydroxy, $C_1$-$C_5$ Alkoxy oder $C_1$-$C_5$ Alkylthio, einen Phenyl- oder einen $C_5$-$C_7$ Cycloalkylring substituiert sein kann, wobei der Phenylring gegebenenfalls durch $C_1$-$C_5$ Alkyl, $C_1$-$C_5$ Alkoxy, Hydroxy oder Halogen substituiert sein kann, einen $C_5$-$C_7$ Cycloalkyl- oder den Phenylrest,

$R_3$ = Wasserstoff, niederes geradkettiges oder verzweigtes $C_1$-$C_5$ Alkyl, das gegebenenfalls durch Hydroxy, $C_1$-$C_5$ Alkoxy, $C_1$-$C_5$ Alkylthio, Mercapto oder Phenyl, substituiert sein kann, oder gebenenfalls durch Hydroxy oder $C_1$-$C_5$ Alkoxy substuiertes Phenyl,

$R_4$, $R_6$, $R_8$ und $R_9$ jeweils unabhängig voneinander Wasserstoff oder $C_1$-$C_5$ Alkyl,

$R_6$ und $R_7$ jeweils unabhängig voneinander Wasserstoff, $C_1$-$C_5$ Alkyl oder gegebenenfalls durch Hydroxy oder $C_1$-$C_5$ Alkoxy substituiertes Phenyl,

X = Wasserstoff, OH oder die Gruppe -$NR_{10}$, $R_{11}$, wobei $R_{10}$ und $R_{11}$ jeweils unabhängig voneinander Wasserstoff oder $C_1$-$C_5$ Alkyl sein soll,

m bzw. n = 0 oder 1

bedeuten, wobei

$R_3$ und $R_4$ zusammen mit dem Kohlenstoffatom, and das sie gebunden sind, einen Fünf- oder Sechsring,

$R_4$ und $R_6$ zusammen mit den Kohlenstoffatomen, an die sie gebunden sind, einen Fünf- oder Sechsring,

$R_6$ und $R_6$ zusammen mit dem Kohlenstoff, an das sie gebunden sind, einen Fünf- oder Sechsring,

$R_7$ und $R_8$ zusammen mit dem Kohlenstoffatom, an das sie gebunden sind, einen Fünf- oder Sechsring bilden können,

sowie deren pharmakologisch unbedenkliche Salze und optische Isomer.

Unter $C_1$-$C_5$ Alkyl sind vorzugsweise die Methyl-, Ethyl-, Isopropyl und Isobutylgruppe zu verstehen. $C_1$-$C_5$ Alkoxy- bzw. Alkylthio sollen bevorzugt die Methoxy- bzw. Methylthiogruppe sein.

Bei dem $C_5$-$C_7$ Cycloalkylrest handelt es sich bevorzugt um den Cyclohexylrest.

Halogen soll insbesondere Chlor oder Brom darstellen.

Bei der bei $R_1$ und $R_2$ angeführten Alkylkette mit 1-9 Kohlenstoffatomen handelt es sich vorzugsweise um die Methyl-, Ethyl-, Isopropyl-, Isobutyl-, sec-Butyl-, n-Pentyl-, n-Nonyl, Allyl- und Methallylgruppe.

Unter der durch einen gegebenenfalls subtituierten Phenylring substituierten Alkylgruppe versteht man insbesondere eine Benzylgruppe.

Bei Gruppe -$NR_{10}R_{11}$ handelt es sich bevorzugt um die Amino-, Dimethylamino- bzw. Diethylaminogruppe.

Für den Fall, daß $R_3$ und $R_4$ bzw. $R_5$ und $R_6$ bzw. $R_7$ und $R_8$ mit dem Kohlenstoffatom, an das sie gebunden sind einen Ring bilden, ist dies bevorzugt der Spiro-Cyclopentylring.

Bilden $R_4$ und $R_6$ zusammen mit den C-Atomen an die sie gebunden sind, einen Ring, so handelt es sich um den Cyclohexyl- oder Cyclopentylring.

X stellt bevorzugt Wasserstoff oder Hydroxyl dar.

Bevorzugte Verbindungen der Formel I sind Verbindungen, in denen $R_1$ Wasserstoff oder Methyl, $R_2$ Wasserstoff oder Methyl, $R_3$ Wasserstoff oder $C_1$ - $C_5$-Alkyl, $R_4$ Wasserstoff oder Methyl, $R_5$ Wasserstoff oder Methyl, $R_5$ Wasserstoff, $R_7$ Wasserstoff, $R_8$ Wasserstoff, $R_9$ Wasserstoff,m die Zahl 0 oder 1, n die Zahl 0 und X eine Hydroxylgruppe bedeuten,

wobei $R_4$ und $R_5$ zusammen mit den C-Atomen an das sie gebunden sind, einen Cyclohexyl-Ring und $R_5$ und $R_5$ zusammen mit dem C-Atom , an das sie gebunden sind, einen Spiro-cyclopentan-Ring darstellen.

Asymmetrische Kohlenstoffatome können die R- oder S-Konfiguration besitzen und die Verbindungen können in optisch aktiver Form oder als racemisches Gemisch vorliegen. Sie sind ebenfalls Gegenstand der Erfindung.

Verbindungen der allgemeinen Formel I werden nach an sich bekannten Verfahren dargestellt, vorzugsweise, indem man

I. eine Verbindung der allgemeinen Formel II

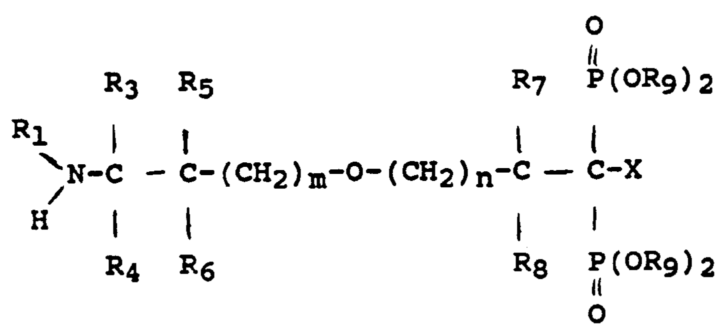

(II),

in der $R_1$, $R_3$-$R_9$, X, m und n die oben angegebenen Bedeutungen haben, mono- oder dialkyliert und gegebenenfalls die entstandenen Tetraester zu Diestern oder Säuren der allgemeinen Formel I verseift,

oder

II. für den Fall, daß X in der allgemeinen Formel I OH bedeutet,

a) eine Carbonsäure der allgemeinen Formel III

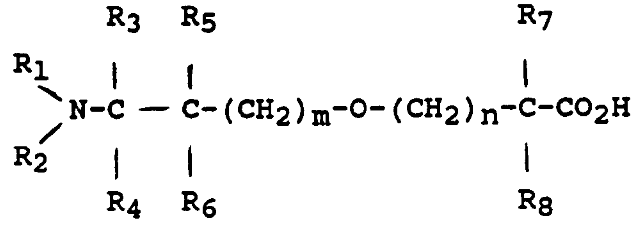

(III),

in der $R_1$-$R_5$, m und n die oben angegebenen Bedeutungen haben, mit einem Gemisch aus phosphoriger Säure oder Phosphorsäure und einem Phosphorhalogenid bzw. Phosphoroxyhalogenid umsetzt und anschließend zur freien Diphosphonsäure verseift,

oder

b) ein Carbonsäurechlorid der allgemeinen Formel IV

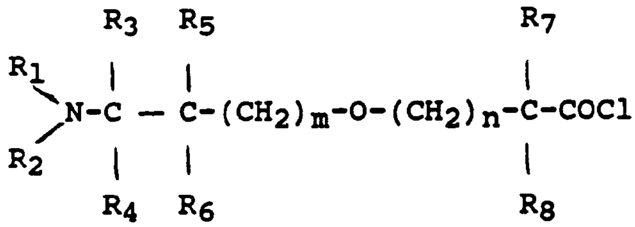

(IV),

in der $R_1$-$R_8$, m und n die oben genannten Bedeutungen haben, wobei $R_1$ auch eine Acylgruppe oder mit $R_2$ zusammen auch als Schutzgruppe den Phthaloylrest darstellen kann, mit einem Trialkylphosphit der allgemeinen Formel V

$P(OR')_3$ (V),

in der R′ für Alkylreste mit 1-4 Kohlenstoffatomen, vorzugsweise Methyl, Ethyl, Isopropyl und Isobutyl steht, zu einem Acylphosphonat der allgemeinen Formel VI

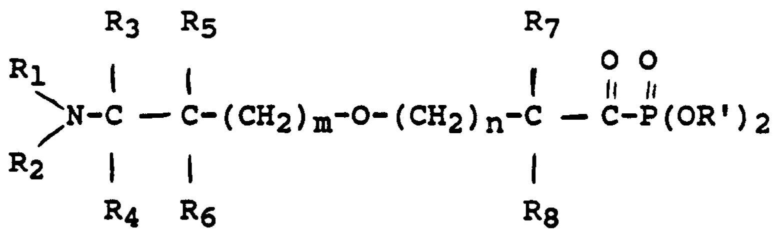

(VI),

in der $R_1$-$R_8$, m, n und R′ die oben genannten Bedeutungen haben, $R_1$ auch eine Acylgruppe oder mit $R_2$ zusammen auch den Phthaloylrest darstellen kann, umsetzt, anschließend mit einem Dialkylphosphit der allgemeinen Formel VII

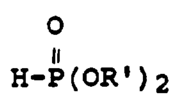

(VII),

in der R′ die oben angegebene Bedeutung hat, zu einem Diphosphonat der allgemeinen Formel VIII

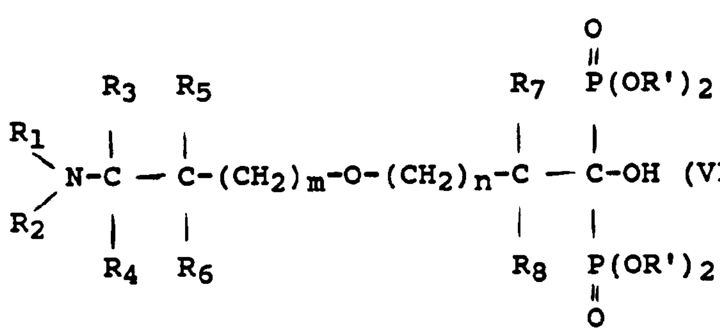

(VIII),

in der $R_1$-$R_8$, m, n, und R′ die oben angebenen Bedeutungen haben, $R_1$ auch eine Acylgruppe oder mit $R_2$ zusammen auch den Phthaloylrest darstellen kann, reagieren läßt und gegebenenfalls die Phthaloylgruppe durch Hydrazinoylse entfernt und die entstandenen Tetraester zu Diestern oder Säuren der allgemeinen Formel I verseift, wobei unter diesen Bedingungen die als Schutzgruppe verwendete Acyl- bzw. Phthaloylgruppe gleichzeitig abgespalten wird,

oder

c) für den fall, daß n = O bedeutet, eine Verbindung der allgemeinen Formel IX

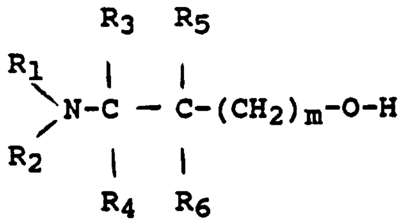

(IX),

in der $R_1$-$R_6$ und m die oben angegebenen Bedeutungen haben mit einem Epoxid der allgemeinen Formel X

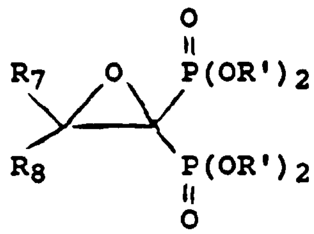

(X),

in der $R_7$, $R_8$ und R′ die oben angegebenen Bedeutungen haben, reagieren läßt und das entstandene. Diphosphonsäurederivat der allgemeinen Formel XI

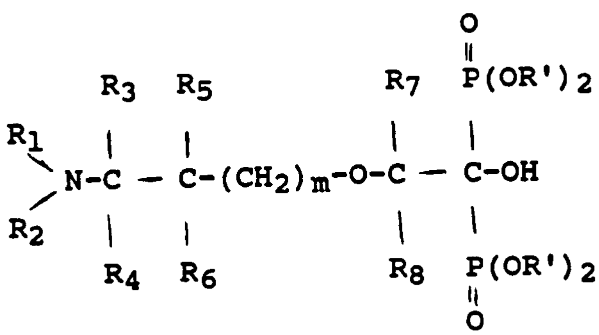

(XI),

gewünschtenfalls zu Diestern oder Säuren verseift,

oder

III. für den Fall, daß X in der allgemeinen Formel I die Gruppe $-NR_{10}R_{11}$ bedeutet, ein Carbonsäurederivat der allgemeinen Formel XII

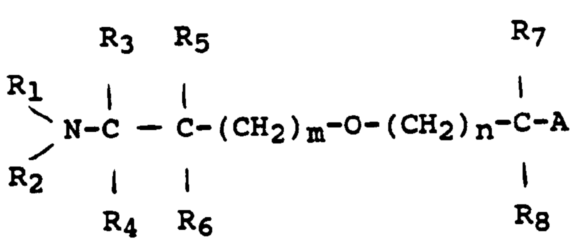

(XII),

in der $R_1$-$R_8$, m und n die oben angegebenen Bedeutungen haben und A eine Nitril-, Iminoether- oder eine $-CONR_{10}R_{11}$-Gruppe, wobei $R_{10}$ und $R_{11}$ die oben angegebenen Bedeutungen haben, darstellt, mit einer Phosphorverbindung der allgemeinen Formel XIII

$PT_3$ (XIII),

in der T = Halogen, OH oder OR′ bedeutet, wobei R′ die oben angegebene Bedeutung hat, umsetzt und gegebenenfalls anschließend verseift,

oder

IV. für den Fall, daß X in der allgemeinen Formel I Wasserstoff bedeutet,

a) eine Verbindung der allgemeinen Formel XIV

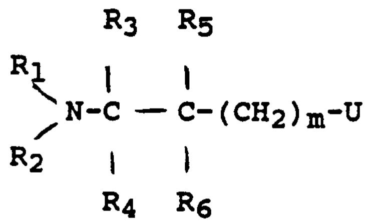

(XIV),

in der $R_1$-$R_6$ und m die oben angegebenen Bedeutungen haben, wobei $R_1$ auch ein Acyl- oder mit $R_2$ zusammen der Phthaloylrest sein kann, und U eine reaktive Gruppe wie z.B. Halogen oder ein Sulfonat darstellt, mit einem Diphosphonsäurederivat der allgemeinen Formel XV,

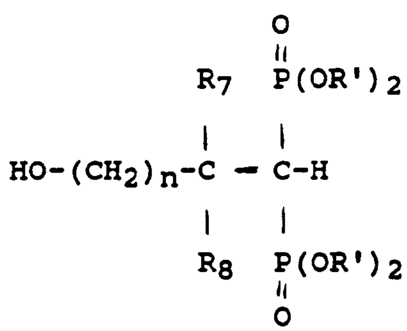

(XV),

in der $R_7$, $R_6$, R′und n die oben angegebenen Bedeutungen haben, reagieren läßt, und die Phthaloylgruppe gewünschtenfalls durch Hydrazinolyse entfernt und die gebildeten Tetraester gegebenenfalls zu Diestern oder Säuren verseift, wobei unter diesen Bedingungen die als Schutzgruppe verwendete Acyl- oder Phthaloylgruppe gleichzeitig abgespalten wird,

oder

b) eine Verbindung der allgemeinen Formel IX

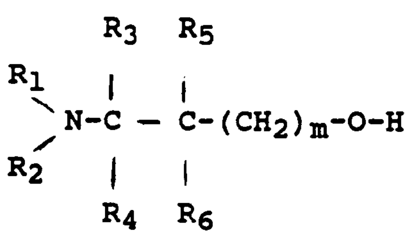

(IX),

in der $R_1$-$R_6$, und m die oben angegebenen Bedeutungen haben, an eine Verbindung der allgemeinen Formel XVI

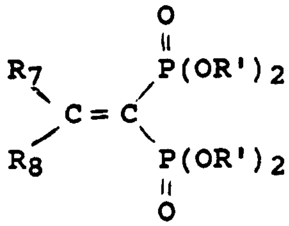
(XVI),

in der $R_7$, $R_8$ und R′ die oben angegebenen Bedeutungen haben, addiert und die entstehenden Tetraester gegebenenfalls zu Diestern oder Säuren verseift,

oder

c) eine Verbindung der allgemeinen Formel XVII

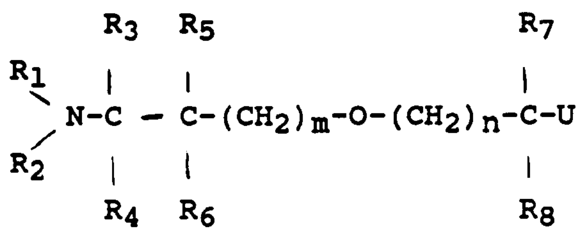
(XVII),

in der $R_1$-$R_8$, U, m und n die oben angegebenen Bedeutungen haben, wobei $R_1$ auch ein Acyl- oder mit $R_2$ zusammen der Phthaloylrest sein kann, mit einem Diphosphonsäurederivat der allgemeinen Formel XVIII

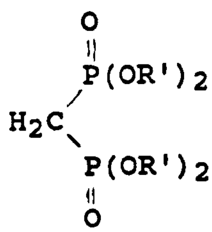
(XVIII),

in der R′ die oben angegebene Bedeutung hat, umsetzt, die Phthaloylgruppe gewünschtenfalls durch Hydrazinolyse entfernt und die entstandenen Tetraester gegebenenfalls zu Diestern oder Säuren verseift, wobei unter diesen Bedingungen die als Schutzgruppe verwendete Acyl- oder Phthaloylgruppe gleichzeitig abgespalten wird

oder

für den Fall, daß $R_8$ Wasserstoff bedeutet,

d) eine Verbindung der allgemeinen Formel XIX

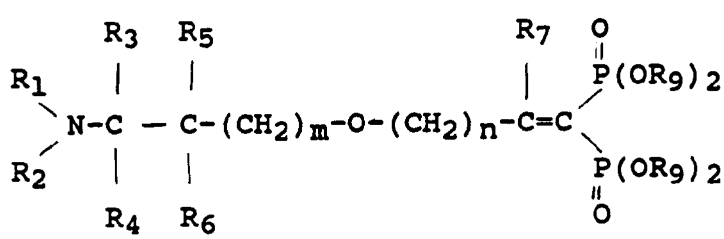
(XIX),

in der $R_1$-$R_7$, $R_9$, m und n die oben angegebenen Bedeutungen haben, wobei $R_1$ auch eine Acylgruppe darstellen kann, katalytisch hydriert

und anschließend gegebenenfalls die entstandenen Tetraester zu Diester oder Säuren verseift, wobei dabei

auch eine evtl. vorhandene Acylgruppe mit abgespalten werden kann und die freien Säuren in pharmakologisch unbedenkliche Salze überführt.

Bei der reduktiven Alkylierung (Verfahren I) behandelt man ein Gemisch aus primärem oder sekundärem Amin der allgemeinen Formel II und einer Carbonylverbindung oder deren Acetal in Gegenwart eines Hydrierungskatalysators, wie Palladium auf Kohle, oder Nickel, mit Wasserstoff unter Atmosphären- oder erhöhtem Druck oder man setzt als Reduktionsmittel Ameisensäure zu. Schließlich lassen sich Alkylierungen eines sekundären Amins der allgemeinen Formel II besonders vorteilhalft nach dem Phasentransferverfahren mit Dialkylsulfaten durchführen.

Die bei Verfahren II a) eingesetzten Carbonsäuren der allgemeinen Formel III werden mit 1-2, vorzugsweise 1.5 mol phosphoriger Säure oder Phosphorsäure und 1-2, vorzugsweise 1.5 mol Phosphortrihalogenid oder Phosphoroxyhalogenid bei Temperaturen von 80°-130°C, vorzugsweise 100-110°C umgesetzt. Man kann die Reaktion auch in Gegenwart von Verdünnungsmitteln wie Halogenkohlenwasserstoffen, insbesondere Chlorbenzol, Tetrachlorethan oder auch Sulfolan bzw. Dioxan durchführen. Die anschließende Hydrolyse erfolgt durch Kochen mit Wasser, zweckmäßigerweise jedoch mit halbkonzentrierter Salz- oder Bromwasserstoffsäure. Als Phosphortrihalogenide kommen in dem gennanten Verfahren beispielsweise Phosphortrichlorid oder Phosphortribromid, als Phosphoroxyhalogenid vor allem Phosphoroxychlorid infrage.

Bei Verfahren II b) läßt man das Säurechlorid der allgemeinen Formel IV mit dem Trialkylphosphit der allgemeinen Formel V bei Temperaturen zwischen 0 und 60°C, vorzugsweise bei 20-40°C zur Reaktion kommen. Man kann ohne Lösungsmittel oder auch in Gegenwart von inerten Lösungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel VI kann isoliert oder direkt weiter umgesetzt werden. Die anschließende Reaktion führt man in Gegenwart einer schwachen Base, vorzugsweise einen sec. Amin wie z.B. Dibutylamin bei einer Temperatur von 0-60°C, vorzugsweise bei 10-30°C durch. Für den Fall, daß $R_1$ und $R_2$ zusammen als Schutzgruppe den Phthaloylrest bilden, wird dieser Rest durch Hydrazinolyse bzw. saure Hydrolyse abgespalten. Bei der Hydrazinolyse setzt man Hydrazin in Essigsäure oder auch Ethanol bei Temperaturen zwischen 20 und 80° ein. Die saure Hydrolyse kann sehr gut durch Kochen mit halbkonzentrierter Salzsäure durchgeführt werden. Auf diese Weise wird auch eine als Schutzgruppe verwendete Acylgruppe - vorzugsweise die Acetylgruppe - abgespalten.

Bei Verfahren II c werden die Alkohole der allgemeinen Formel IX in der Regel in Form ihrer Alkalisalze, vorzugsweise als Natriumsalze eingesetzt. Als Lösungsmittel verwendet man bevorzugt Toluol, Dioxan, Tetrahydrofuran oder auch Dimethylformamid; die Reaktionen werden zwischen 20 und 80°C durchgeführt.

Bei Verfahren III setzt man die Nitrile der allgemeinen Formal XII mit phosphoriger Säure bei Temperaturen von 110-180°C um. Die Reaktion kann ohne oder in Gegenwart von aprotischen Lösungsmitteln wie z.B. Diethylenglykoldimethylether oder Diethylenglykoldiethylether durchgeführt werden. Man kann die Nitrile jedoch auch mit einem Phosphortrihalogenid, z.B. Phosphortribromid oder Phosphortrichlorid in einem inerten Lösungsmittel wie z.B. Dioxan oder Tetrahydrofuran gegebenenfalls unter Zusatz von Wasser bei Temperaturen von 20-80°C zur Reaktion bringen. Iminoether der allgemeinen Formel XII läßt man mit Dialkylphosphiten vorzugsweise in Gegenwart äquimolarer Mengen Natrium in inerten Lösungsmitteln wie Diethylether, Dioxan oder auch Benzol reagieren, wobei die Umsetzungen in der Regel bei der Rückflußtemperatur des entsprechenden Lösungsmittels stattfindet. Säureamide der allgemeinen Formel XII kann man in inerten Lösungsmitteln wie z.B. halogenierten Kohlenwasserstoffen oder Ethern wie z.B. Diethylether mit einem Gemisch aus Phosphorpentahalogenid/phosphoriger Säure oder auch Oxalylchlorid/Trialkylphosphit umsetzen.

Bei Verfahren IV a) setzt man das Diphosphonsäurederivat der allgemeinen Formel XV in Form eines Natrium- oder Kaliumsalzes ein. Hierzu wird es mit Natrium, Kalium oder dem entsprechenden Hydrid in einem inerten Lösungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reatkion gebracht. Die Temperatur liegt hierbei bei 20-110°C.

Falls $R_1$ und $R_2$ zusammen als Schutzgruppe den Phthaloylrest bilden oder $R_1$ eine Acylgruppe, vorzugsweise die Acetylgruppe darstellt, werden diese Reste wie in Verfahren II b beschrieben abgespalten.

Bei Verfahren IV b werden die Alkohole der allgemeinen Formel IX in Form ihrer Alkalisalze, vorzugsweise der Natriumsalze eingesetzt. Hierzu werden sie mit Natrium bzw. Natriumhydrid in einem inerten Lösungsmittel wie Benzol, Toluol, Dioxan oder Dimethylformamid bei einer Temperatur von 0-60°C, vorzugsweise bei 25°C umgesetzt. Das Alkalisalz wird in der Regel ohne Isolierung mit dem entsprechenden Diphosphonat der allgemeinen Formel XVI zur Reaktion gebracht. Die Temperatur liegt bei 20-80°C.

Bei Verfahren IV c) setzt man den Methylendiphosphonsäureester der allgemeinen Formel XVIII in Form seines Natrium- oder Kaliumsalzes ein. Hierzu wird er mit Natrium, Kalium oder dem entsprechenden Hydrid in einem inerten Lösungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20-110°C.

Die Hydrierung bei Verfahren IV d wird in Gegenwart eines Edelmetallkatalysators wie z.B. Palladium auf Kohle oder Platin in einem Alkohol wie Methanol oder Ethanol als Lösungsmittel oder auch in Wasser durchgeführt. Man kann jedoch auch Nickel in alkalischem Medium verwenden. Die Abspaltung der N-Acylgruppe kann alkalisch, vorzugsweise jedoch sauer mit z.B. 6N Salzsäure vorgenommen werden.

Optisch aktive Verbindungen der Formel I werden in der Regel in der Weise hergestellt, daß man optisch aktive Ausgangsverbindungen einsetzt.

Die bei Verfahren II a eingesetzten Amino-oxa-alkancarbonsäuren werden in der Regel auf folgende Weise hergestellt.

Das entsprechende Aminoalkanol wird z.B. mit einem Halogenessigsäureester zu einem Amino-oxa-alkancarbonsäureester umgesetzt, der abhängig von der Kettenlänge und der Substitution am Stickstoffatom zu einem Oxalactam cyclisieren kann. Der entstandene Carbonsäureester wird nach üblichen Methoden sauer oder alkalisch verseift. Im Falle der Ringbildung wird das Lactam durch Kochen mit Bariumhydroxidlösung geöffnet und das Bariumsalz der Amino-oxa-alkancarbonsäure mit Schwefelsäure in die freie Säure überführt.

Die bei diesem Verfahren sowie auch Verfahren II c und IV b eingesetzten Aminoalkanole sind in der Regel literaturbekannt oder lassen sich aus den entsprechenden Aminosäuren bzw. deren Ester leicht durch Reduktion mit z.B. Lithiumaluminiumhydrid herstellen.

Die bei Verfahren III verwendeten Amino-oxa-alkancarbonsäurenitrile oder Amide der Formel XII lassen sich aus den entsprechenden Aminoalkanolen der Formel IX durch Umsetzung mit Halogenessigsäurenitrilen bzw. Halogenessigsäureamiden synthetisieren. Aus den so gewonnen Nitrilen kann man nach üblichen Verfahren, z.B. durch Reaktion mit einem niederen Alkohol in Gegenwart von gasförmigem Chlorwasserstoff die entsprechenden Iminoether erhalten.

Durch Reaktion eines Aminoalkanols mit einem Phosphorhalogenid wie z.B. Phosphortrichlorid oder Phosphortribromid bzw. mit einem aliphatischen oder aromatischen Sulfochlorid, wie z.B. Methansulfochlorid oder Benzolsulfochlorid erhält man die bei Verfahren IV a eingesetzten Verbindungen der allgemeinen Formel XIV.

Die bei Verfahren IV b eingesetzten Verbindungen der allgemeinen Formel XIX können z.B. aus einer Verbindung der Formel I durch Eliminierung einer H-X-Gruppe hergestellt werden, wobei z.B. ein Halogen, vorzugsweise Brom oder Chlor, oder eine Acyloxygruppe, insbesondere die Acetoxy- oder Propionyloxygruppe, darstellt. Die Eliminierung kann durch Basen wie z.B. tert.-Amine, insbesondere Triethylamin, Pyridin oder Diazabicycloundecen, in inerten Lösungsmitteln wie Alkoholen, Ethern (z.B. Dioxan oder Tetrahydrofuran) erfolgen. Bei der Abspaltung von Essigsäure bzw. Propionsäure setzt man vorzugsweise das Tetranatrium- oder Tetrakaliumsalz der entsprechenden Diphosphonsäure ein und führt die Abspaltung durch Erhitzen auf 180-300°C, vorzugsweise 180-240°C durch. Aus dem Tetraalkalisalz kann man die freien Säuren dann z.B. durch Behandlung mit einem sauren Ionenaustauscher (z.B. Amberlite-IR 120, $H^+$-Form) freisetzen.

Die oben angeführten Ausgangsverbindungen können als Racemate oder als Enaniomere eingesetzt werden, wobei die optisch aktiven Verbindungen üblicherweise aus entsprechend optisch aktiven Aminosäuren erhalten werden.

Die bei den Verfahren gegebenenfalls anfallenden Tetraalkylester können zu Diestern oder den freien Tetrasäuren verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung zu freien Diphosphonsäuren geschieht in der Regel durch Kochen mit halbkonzentrierter Salz- oder Bromwasserstoffsäure. Man kann jedoch auch eine Spaltung mit Trimethylsilylhalogenid, vorzugsweise dem Bromid oder Jodid vornehmen. Die freien Diphosphonsäuren können umgekehrt durch Kochen mit Orthoameisensäurealkylestern wieder in die Tetraalkylester überführt werden. Die freien Diphosphonsäuren der allgemeinen Formel I können als freie Säuren oder in Form ihrer Mono- oder Dialkalisalze isoliert werden. Die Alkalisalze lassen sich in der Regel durch Umfällen aus Wasser/Methanol oder Wasser/Aceton gut reinigen.

Als pharmakologisch verträgliche Salze werden vor allem Alkali- oder Ammoniumsalze verwendet, die man in üblicher Weise z. B. durch Titrieren der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl- oder Triethylamin herstellt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten. Die Dosierung kann von verschiedenen Faktoren, wie Applikationsweise, Spezies, Alter und/oder individuellem Zustand abhängen. Die tägliche zu verabreichenden Dosen liegen bei etwa 0.1-100 mg/Mensch, vorzugsweise bei 1-20 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonate, sowie deren Natriumsalze, Methyl-, Ethyl- oder Isopropylester:

5-N,N-Dimethylamino-3-oxa-pentan-1-hydroxy-1,1-diphosphonsäure
5-N-Methyl-N-propylamino-3-oxa-pentan-1-hydroxy-1,1-diphosphonsäure
5-N-Methyl-N-nonylamino-3-oxa-pentan-1-hydroxy-1,1-diphosphonsäure
5-N-Benzylamino-3-oxa-pentan-1-hydroxy-1,1-diphosphonsäure
5-N-Isobutyl-N-methylamino-3-oxa-pentan-1-hydroxy-1,1-diphosphonsäure
5-N-Methallyl-N-methylamino-3-oxa-pentan-1-hydroxy-1,1-diphosphonsäure
5-N-(2-Methoxyethyl)-N-methylamino-3-oxa-pentan-1-hydroxy-1,1-diphosphonsäure
5-N-(2-Hydroxyethyl)-N-methylamino-3-oxa-pentan-1-hydroxy-1,1-diphosphonsäure
5-N-(2-Methylmercaptoethyl)-N-methylamino-3-oxa-pentan-1-hydroxy-1,1-diphosphonsäure
5-N-(4-Methylbenzyl)amino-3-oxa-pentan-1-hydroxy-1,1-diphosphonsäure
5-N-(2-Chlorbenzyl)amino-3-oxa-pentan-1-hydroxy-1,1-diphosphonsäure
5-N-Cyclohexyl-N-methylamino-3-oxa-pentan-1-hydroxy-1,1-diphosphonsäure
5-Amino-7-methyl-3-oxa-octan-1-hydroxy-1,1-diphosphonsäure
5-Amino-6-phenyl-3-oxa-hexan-1-hydroxy-1,1-diphosphonsäure
5-Amino-6-(3-indolyl)-3-oxa-hexan-1-hydroxy-1,1-diphosphonsäure
5-Amino-6-(4-imidazolyl)-3-oxa-hexan-1-hydroxy-1,1-diphosphonsäure
5-Amino-6-hydroxy-3-oxa-hexan-1-hydroxy-1,1-diphosphonsäure
5-Amino-7-methylmercapto-3-oxa-heptan-1-hydroxy-1,1-diphosphonsäure
5-N-Methyl-N-propylamino-3-oxa-hexan-1-hydroxy-1,1-diphosphonsäure
5-N-Methyl-N-pentylamino-3-oxa-hexan-1-hydroxy-1,1-diphosphonsäure
5-N-Allyl-N-methylamino-3-oxa-hexan-1-hydroxy-1,1-diphosphonsäure
5-Amino-6-(4-hydroxyphenyl)-3-oxa-hexan-1-hydroxy-1,1-diphosphonsäure
5-Amino-4,4-dimethyl-3-oxa-pentan-1-hydroxy-1,1-diphosphonsäure
5-Amino-4-phenyl-3-oxa-pentan-1-hydroxy-1,1-diphosphonsäure
5-Amino-2-methyl-3-oxa-pentan-1-hydroxy-1,1-diphosphonsäure-
5-Amino-2,2-dimethyl-3-oxa-pentan-1-hydroxy-1,1-diphosphonsäure
5-Amino-2-phenyl-3-oxa-pentan-1-hydroxy-1,1-diphosphonsäure
5-Amino-5,5-butylen-3-oxa-pentan-1-hydroxy-1,1-diphosphonsäure
5-N,N-Dimethylamino-5,5-pentylen-3-oxa-pentan-1-hydroxy-1,1-diphosphonsäure
4-(2-Aminocyclohexyl)-3-oxa-butan-1-hydroxy-1,1-diphosphonsäure
3-(2-Aminocyclopentyl)-3-oxa-propan-1-hydroxy-1,1-diphosphonsäure
4-(2-Aminocyclopentyl)-3-oxa-butan-1-hydroxy-1,1-diphosphonsäure
4-(2-N,N-Dimethylamino-cyclohexyl)-3-oxa-butan-1-hydroxy-1,1-diphosphonsäure
5-Amino-4,4-butylen-3-oxa-pentan-1-hydroxy-1,1-diphosphonsäure
6-Amino-2,2-butylen-3-oxa-hexan-1-hydroxy-1,1-diphosphonsäure
5-N,N-Dimethylamino-2,2-pentylen-3-oxa-pentan-1-hydroxy-1,1-diphosphonsäure
1,5-Diamino-3-oxa-pentan-1,1-diphosphonsäure
5-Amino-1-N,N-diethylamino-3-oxa-hexan-1,1-diphosphonsäure
5-Amino-3-oxa-hexan-1,1-diphosphonsäure
R-5-Amino-3-oxa-hexan-1,1-diphosphonsäure
S-5-Amino-3-oxa-hexan-1,1-diphosphonsäure
5-N,N-Dimethylamino-3-oxa-pentan-1,1-diphosphonsäure
R-5-Amino-7-methyl-3-oxa-octan-1-hydroxy-1,1-diphosphonsäure
R-5-Amino-6-phenyl-3-oxa-hexan-1-hydroxy-1,1-diphosphonsäure
S-5-Amino-6-phenyl-3-oxa-hexan-1-hydroxy-1,1-diphosphonsäure

R-5-Amino-1.6-dihydroxy-3-oxa-hexan-1,1-diphosphonsäure
S-5-Amino-1.6-dihydroxy-3-oxa-hexan-1,1-diphosphonsäure
R-5-Amino-7-methylmercapto-3-oxa-heptan-1-hydroxy-1,1-diphosphonsäure
S-5-Amino-7-methylmercapto-3-oxa-heptan-1-hydroxy-1,1-diphosphonsäure
R-5-N-Methyl-N-propylamino-3-oxa-hexan-1-hydroxy-1,1-diphosphonsäure
S-5-N-Methyl-N-propylamino-3-oxa-hexan-1-hydroxy-1,1-diphosphonsäure
R-5-N-Methyl-N-pentylamino-3-oxa-hexan-1-hydroxy-1,1-diphosphonsäure
S-5-N-Methyl-N-pentylamino-3-oxa-hexan-1-hydroxy-1,1-diphosphonsäure
R-5-N-Allyl-N-methylamino-3-oxa-hexan-1-hydroxy-1,1-diphosphonsäure
S-5-N-Allyl-N-methylamino-3-oxa-hexan-1-hydroxy-1,1-diphosphonsäure
R-5-Amino-6-(4-hydroxyphenyl)-3-oxa-hexan-1-hydroxy-1,1-diphosphonsäure
S-5-Amino-6-(4-hydroxyphenyl)-3-oxa-hexan-1-hydroxy-1,1-diphosphonsäure
R-5-Amino-4-phenyl-3-oxa-pentan-1-hydroxy-1,1-diphosphonsäure
S-5-Amino-4-phenyl-3-oxa-pentan-1-hydroxy-1,1-diphosphonsäure
R-5-Amino-2-methyl-3-oxa-pentan-1-hydroxy-1,1-diphosphonsäure
S-5-Amino-2-methyl-3-oxa-heptan-1-hydroxy-1,1-diphosphonsäure
R-5-Amino-2-phenyl-3-oxa-pentan-1-hydroxy-1,1-diphosphonsäure
S-5-Amino-2-phenyl-3-oxa-pentan-1-hydroxy-1,1-diphosphonsäure
5-Amino-2-methyl-3-oxa-hexan-1-hydroxy-1,1-diphosphonsäure
6-Amino-3-oxa-heptan-1-hydroxy-1,1-diphosphonsäure
6-Amino-5-methyl-3-oxa-hexan-1-hydroxy-1,1-diphosphonsäure
6-Amino-4-methyl-3-oxa-hexan-1-hydroxy-1,1-diphosphonsäure

Die nachfolgenden Beispiele zeigen einige der Verfahrensvarianten, die zur Synthese der erfindungsgemäßen Verbindungen verwendet werden können. Sie sollen jedoch nicht eine Einschränkung des Erfindungsgegenstandes darstellen. Die Verbindungen fallen in der Regel als hochschmelzende Festprodukte an (Mono- oder Dinatriumsalz), deren Struktur durch H-, P- und gegebenenfalls durch $^{13}C$ NMR-Spektroskopie gesichert wurde. Die Reinheit der Substanzen wurde mittels C,H,N,P,S, Na-Analyse sowie durch Dünnschichtelektrophorese (Cellulose, Oxalat-Puffer von pH = 4.0) bestimmt. Zur Charakterisierung der einzelnen Verbindungen werden die $M_{rel}$-Werte (= relative Mobilität) bezogen auf Pyrophosphat ($M_{rel}$ = 1) angegeben.

## Beispiel 1

**R,S-5-Amino-3-oxa-hexan-1-hydroxy-1,1-diphosphonsäure**

0.67 g (5 mmol) R,S-5-Amino-3-oxa-hexansäure werden mit 0.82 g (10 mmol) phosphoriger Säure bei 100°C geschmolzen. Man entfernt das Ölbad, tropft 1 ml (11 mmol) Phosphortrichlorid zu und erhitzt weitere 24 h auf 100°C Außentemperatur. Nach dem Abkühlen versetzt man mit 10 ml Wasser, kocht 45 min unter Rückfluß, saugt ab, engt das Filtrat auf die Hälfte ein, stellt die Lösung mit 10 N Natronlauge auf pH =5, versetzt mit 20 ml Methanol und kühlt die Lösung im Eisbad. Der ausgefallene Niederschlag wird abgesaugt, mit Methanol gewaschen und getrocknet. Der Rückstand wird in wenig Wasser gelöst und über eine Ionenaustauschersäule (35 gAmberlite-IR 120; $H^+$-Form) gereinigt. Man erhält 0.49 g = 34 % der gewünschten Verbindung, die 0.5 mol Wasser enthält; Fp: 240-260°C; $M_{rel}$: 0.40.

Die als Ausgangsmaterial verwendete R,S-5-Amino-3-oxahexansäure wurde auf folgende Weise hergestellt: R,S-5-Methyl-morpholin-3-on (Fp: 62-64°C) wird mit Bariumhydroxid gekocht und aus dem Bariumsalz mit Schwefelsäure bei pH = 5 die freie Säure (Fp: 190-193°C) hergestellt.

In analoger Weise erhält man durch Umsetzung von phosphoriger Säure und Phosphortrichlorid mit

a) R,S-5-N,N-Dimethylamino-3-oxa-hexansäure (Fp: 108-110°C) (hergestellt durch reduktive Methylierung von R,S-5-Amino-3-oxa-hexansäure mittels Ameisensäure/Formaldehyd) die R,S-5-N,N-Dimethylamino-3-oxa-hexan-1-hydroxy-1,1-diphosphonsäure als freie Säure mit 1 mol Wasser in einer Ausbeute von 36 %; Fp: ca. 270°C $M_{rel}$: 0.40.

## Beispiel 2

Analog wie im Beispiel 1 beschrieben erhält man durch Verwendung von

a) 5-Amino-3-oxa-pentansäure (Fp: 188-190°C) die 5-Amino-3-oxa-pentan-1-hydroxy-1.1-diphosphonsäure mit 1 mol Wasser in einer Ausbeute von 31 %; Fp: 255-260°C; $M_{rel}$: 0.30.

b) 6-(N-Acetyl-amino)-3-oxa-hexansäure (Öl) die 6-Amino-3-oxa-hexan-1-hydroxy-1.1-diphosphonsäure mit 1 mol Wasser in einer Ausbeute von 23 %; Fp: 125-130°C; $M_{rel}$: 0.30.

c) 5-N-Methylamino-3-oxa-pentansäure (Fp: 242-245°C) die 5-N-Methylamino-3-oxa-pentan-1-hydroxy-1.1-diphosphonsäure mit 1 mol Wasser in einer Ausbeute von 28 %; Fp: 155-160°C; $M_{rel}$: 0.35

d) 6-N,N-Dimethylamino-3-oxa-hexansäure-hydrochlorid (Öl) die 6-N,N-Dimethylamino-3-oxa-hexan-1-hydroxy-1.1-diphosphonsäure mit 1 mol Wasser in einer Ausbeute von 22 %; Fp: 115-120°C; $M_{rel}$: 0.30

e) R-5-Amino-3-oxa-hexansäure (Fp: 182-185°C; $[\alpha]_D^{20}$: -30.5°, c = 1.5 in Wasser) die R-5-Amino-3-oxa-hexan-1-hydroxy-1.1-diphosphonsäure mit 1 mol Wasser in einer Ausbeute von 30 %, Fp: 118-123°C; $[\alpha]_D^{20}$: -22.6°, c = 0.8 in Wasser; $M_{rel}$: 0.30.

f) S-5-Amino-3-oxa-hexansäure (Fp: 180-182°C; $[\alpha]_D^{20}$: -28.5°, c = 1.4 in Wasser) die S-5-Amino-3-oxa-hexan-1-hydroxy-1.1-diphosphonsäure mit 1 mol Wasser in einer Ausbeute von 34 %, Fp: 115-120°C; $[\alpha]_D^{20}$: +21.2°, c = 0.8 in Wasser; $M_{rel}$: 0.30.

g) 5-Amino-6-methyl-3-oxa-heptansäure (Öl) die 5-Amino-6-methyl-3-oxa-heptan-1-hydroxy-1.1-diphosphonsäure mit 1 mol Wasser in einer Ausbeute von 22 %, Fp: 135-140°C; $M_{rel}$: 0.35.

h) S-5-Amino-6-methyl-3-oxa-heptansäure (Fp: 140-145°C; $[\alpha]_D^{20}$: +23.9°, c = 1 in Wasser) die S-5-Amino-6-methyl-3-oxa-heptan-1-hydroxy-1.1-diphosphonsäure mit 1 mol Wasser in einer Ausbeute von 27 %, Fp: 245-250°C; $[\alpha]_D^{20}$: +19.3°, c = 1.0 in Wasser; $M_{rel}$: 0.30.

i) R-5-Amino-6-methyl-3-oxa-heptansäure (Fp: 143-147°C; $[\alpha]_D^{20}$: -24.3°, c = 1.1 in Wasser) die R-5-Amino-6-methyl-3-oxa-heptan-1-hydroxy-1.1-diphosphonsäure mit 1 mol Wasser in einer Ausbeute von 26 %, Fp: 245-250°C; $[\alpha]_D^{20}$: -18.9°, c = 1.0 in Wasser; $M_{rel}$ 0.30.

j) S-5-Amino-7-methyl-3-oxa-octansäure (Fp: 148-150°C; $[\alpha]_D^{20}$: +17.7°, c = 1.2 in Wasser) die S-5-Amino-7-methyl-3-oxa-octan-1-hydroxy-1.1-diphosphonsäure mit 1 mol Wasser in einer Ausbeute von 31 %, Fp: 250-255°C; $[\alpha]_D^{20}$: +14.8°, c = 1.2 in Wasser; $M_{rel}$: 0.30.

k) 5-Amino-5-methyl-3-oxa-hexansäure (Fp: 243-245°C) die 5-Amino-5-methyl-3-oxa-hexan-1-hydroxy-1.1-diphosphonsäure mit 1 mol Wasser in einer Ausbeute von 27 %, Fp: 155-160°C; $M_{rel}$: 0.40.

l) 5-Amino-4-methyl-3-oxa-pentansäure (Fp: 213-215°C;) die 5-Amino-4-methyl-3-oxa-pentan-1-hydroxy-1.1-diphosphonsäure mit 1 mol Wasser in einer Ausbeute von 33 %, Fp: 145-150°C; $M_{rel}$: 0.30.

o) 3-(2-Aminocyclohexyl)-3-oxa-propionsäure (Fp: 218-220°C;) die 3-(2-Aminocyclohexyl)-3-oxa-propan-1-hydroxy-1.1-diphosphonsäure mit 1 mol Wasser in einer Ausbeute von 19 %, Fp: 215-220°C; $M_{rel}$: 0.25.

p) 5-Amino-4.4-pentylen-3-oxa-pentansäure (Fp: 203-205°C;) die 5-Amino-4.4-pentylen-3-oxa-pentan-1-hydroxy-1.1-diphosphonsäure mit 1 mol Wasser in einer Ausbeute von 29 %, Fp: 235-240°C; $M_{rel}$: 0.30.

r) R-5-Amino-4-methyl-3-oxa-pentansäure (Fp: 210-212°C; $[\alpha]_D^{20}$ -97.0°, c = 1 in Wasser) die R-5-Amino-4-methyl-3-oxa-pentan-1-hydroxy-1.1-diphosphonsäure mit 1 mol wasser in einer Ausbeute von 23 %, Fp: 140-145°C; $[\alpha]_D^{20}$: -22.5°, c = 1 in Wasser; $M_{rel}$: 0.30.

s) S-5-Amino-4-methyl-3-oxa-pentansäure (Fp: 212-214°C; $[\alpha]_D^{20}$: +97.8°, c = 1 in Wasser) die S-5-Amino-4-methyl-3-oxa-pentan-1-hydroxy-1.1-diphosphonsäure mit 1mol Wasser in einer Ausbeute von 15 %, Fp: 145-150°C; $[\alpha]_D^{20}$: +22.9°, c = 1 in Wasser; $M_{rel}$: 0.30.

Die im Beispiel 2 a eingesetzte 5-Amino-3-oxa-pentansäure wird auf folgende weise hergestellt:

Ethanolamin wird in Gegenwart von Natriumhydrid mit Chloressigsäureethylester zum Morpholin-3-on (Fp: 100-102°C) umgesetzt und daraus durch Erhitzen mit Bariumhydroxid und anschließender Behandlung mit Schwefelsäure die gewünschte Säure erhalten.

In gleicher Weise wurden die in der folgenden Tabelle aufgeführten Zwischenprodukte hergestellt und umgesetzt.

| Beisp.Nr. | Morpholinon | Fp.°C | $[\alpha]_D^{23}$ in Methanol |
|---|---|---|---|
| 2 c | N-Methyl-morpholin-3-on | Öl | - |
| 2 e | R-5-Methyl-morpholin-3-on | 60-62 | -3.7° |
| 2 f | S-5-Methyl-morpholin-3-on | 59-61 | +3.1° |
| 2 g | 5-Isopropyl-morpholin-3-on | 86-88 | - |
| 2 h | S-5-Isopropyl-morpholin-3-on | 86-88 | +3.9° |
| 2 i | R-5-Isobutyl-morpholin-3-on | 87-89 | -4,3° |
| 2 j | S-5-Isobutyl-morpholin-3-on | 70-72 | -4.° |
| 2 k | 5,5-Dimethyl-morpholin-3-on | 133-35 | - |
| 2 l | 6-Methyl-morpholin-3-on | 96-98 | - |
| 2 o | 2-Oxa-5-aza-bicyclo[4.4.0]-decan-4-on | 174-76 | - |
| 2 p | 1-Oxa-4-aza-bicyclospiro-[5.5]undecan-3-on | 93-95 | - |
| 2 r | R-6-Methyl-morpholin-3-on | 96-98 | -134.1° |
| 2 s | S-6-Methyl-morpholin-3-on | 95-97 | +131.1° |

Im Falle der Beispiele 2 b und 2 n wurden die Ausgangsaminoalkohole erst am Stickstoff acetyliert, anschließend in Gegenwart von Natriumhydrid mit Bromessigsäureethylester zum entsprechenden Alkoxiessigsäureethylester umgesetzt und dann mit Natronlauge verseift. Alle Zwischenprodukte fielen als Öle an.

Im Falle der Beispiele 2 d und 2 m wurden die tert.-Aminoalkohole in Gegenwart von Natriumhydrid mit Bromessigsäureethylester (2 m) bzw. dem Natriumsalz der Chloressigsäure (2 d) umgesetzt, im letzteren Fall mit Ethanol-Schwefelsäure zum entsprechenden Ethylester verestert und in beiden Fällen anschließend mit 2 N Salzsäure verseift. Alle Zwischenprodukte fielen auch hier als Öle an.

**Beispiel 3**

5-Amino-3-oxa-pentan-1.1-diphosphonsäure

Zu 144 mg (6 mmol) Natriumhydrid in 5 ml abs. Toluol tropft man 1.73 g (6mmol) Methandiphosphonsäuretetraethylester. Man läßt nach Beendigung der Wasserstoffentwicklung noch 30 min nachrühren und tropft

dann 1.7 g (6 mmol) N-(2-Brommethoxy-ethyl)phthalimid (Fp: 83-85°C) zu. Man läßt 24 h bei Raumtemperatur rühren, versetzt die Mischung mit Wasser, stellt die wässrige Phase mit 2 N Salzsäure auf pH = 5 ein, trennt die organische Phase ab, trocknet und engt ein. Den Rückstand reinigt man über 250 g Kieselgel (Elutionsmittel: Methylenchlorid/Methanol i.V. 4/1) und erhält 0.35 g = 12 % des 5-Phthalimido-3-oxa-pentan-1.1-diphosphonsäure-tetraethylester als ölige Substanz. Der Ester wird anschließend mit 10 ml 6 N Salzsäure 12 h unter Rückfluß gekocht, nach dem Abkühlen wird ausgefallene Phthalsäure abgesaugt, das Filtrat mit Kohle behandelt, filtriert und eingeengt. Den Rückstand nimmt man in Wasser auf, stellt die Lösung mit 2 N Natronlauge auf $P_H$ = 5 und versetzt unter Eiskühlung mit einem großen Überschuß an Methanol. Der Niederschlag wird abgesaugt und getrocknet. Man erhält 0.125 g = 7.2 % der gewünschten Verbindung in Form des Mononatriumsalzes mit 1 mol Wasser, Fp: >300°C; $M_{rel}$: 0.30.

**Patentansprüche**

1. Verbindungen der allgemeinen Formel I

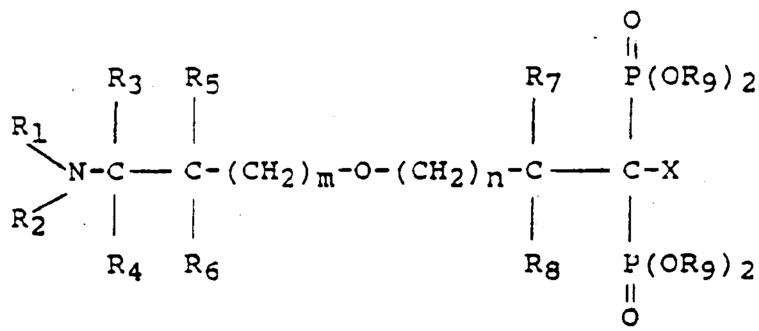

(I).

in der
$R_1$ und $R_2$ jeweils unabhängig voneinander Wasserstoff, eine geradkettige oder verzweigte, gesättigte oder ungesättigte Alkylkette mit 1-9 Kohlenstoffatomen, die gegebenenfalls durch Hydroxy, $C_1$-$C_5$ Alkoxy oder $C_1$-$C_5$ Alkylthio, einen Phenyl- oder einen $C_5$-$C_7$ Cycloalkylring substitutiert sein kann, wobei der Phenylring gegebenenfalls durch $C_1$-$C_5$ Alkyl, $C_1$-$C_5$ Alkoxy, Hydroxy oder Halogen substitutier sein kann, einen $C_5$-$C_7$ Cycloalkyl- oder den Phenylrest,
$R_3$ = Wasserstoff, niederes geradkettiges oder verzweigtes $C_1$-$C_5$ Alkyl, das gegebenenfalls durch Hydroxy, $C_1$-$C_5$ Alkoxy, $C_1$-$C_5$ Alkylthio, Mercapto oder Phenyl substituiert sein kann, oder gegebenenfalls durch Hydroxy oder $C_1$-$C_5$ Alkoxy substituiertes Phenyl,
$R_4$, $R_6$, $R_6$ und $R_9$ jeweils unabhängig voneinander Wasserstoff oder $C_1$-$C_5$ Alkyl,
$R_5$ und $R_7$ jeweils unabhängig voneinander Wasserstoff, $C_1$-$C_5$ Alkyl oder gegebenenfalls durch Hydroxy oder $C_1$-$C_5$ Alkoxy substituiertes Phenyl,
X = Wasserstoff, OH oder die Gruppe -$NR_{10}$, $R_{11}$, wobei $R_{10}$ und $R_{11}$ jeweils unabhängig voneinander Wasserstoff oder $C_1$-$C_5$ Alkyl sein soll,
m bzw. n = 0 oder 1
bedeuten, wobei
$R_3$ und $R_4$ zusammen mit dem Kohlenstoffatom, an das sie gebunden sind, einen Fünf- oder Sechsring,
$R_4$ und $R_6$ zusammen mit den Kohlenstoffatomen, an die sie gebunden sind, einen Fünf- oder Sechsring,
$R_5$ und $R_6$ zusammen mit dem Kohlenstoff, an das sie gebunden sind, einen Fünf- oder Sechsring,
$R_7$ und $R_8$ zusammen mit dem Kohlenstoffatom, an das sie gebunden sind, einen Fünf- oder Sechsring bilden können,
sowie deren pharmakologisch unbedenklichen Salze und optischen Isomere.

2. Verbindungen der Formel I gemäß Anspruch 1, in denen $R_1$ Wasserstoff oder Methyl, $R_2$ Wasserstoff oder Methyl, $R_3$ Wasserstoff oder $C_1$-$C_5$ Alkyl, $R_4$ Wasserstoff oder Methyl, $R_6$ Wasserstoff oder Methyl, $R_6$ Wasserstoff, $R_7$ Wasserstoff, $R_6$ Wasserstoff, $R_9$ Wasserstoff, m die Zahl 0 oder 1, n die Zahl 0 und X eine Hydroxylgruppe bedeuten, wobei $R_4$ und $R_6$ zusammen mit den C-Atomen, an die sie gebunden sind, einen Cyclohexyl-Ring und $R_5$ und $R_6$ zusammen mit dem C-Atom, an das sie gebunden sind, einen Spirocyclo-pentan-Ring darstellen.

**3.** Verfahren zur Herstellung von Verbindungen der allgemeinen Formel I

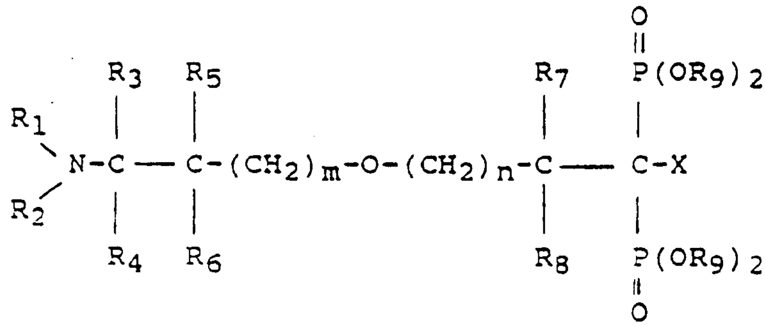

(I),

in der
$R_1$ und $R_2$ jeweils unabhängig voneinander Wasserstoff, eine geradkettige oder verzweigte, gesättigte oder ungesättigte Alkylkette mit 1-9 Kohlenstoffatomen, die gegenbenenfalls durch Hydroxy, $C_1$-$C_5$ Alkoxy oder $C_1$-$C_5$ Alkykthio, einen Phenyl- oder einen $C_5$-$C_7$ Cycloalkylring substituiert sein kann, wobei der Phenylring gegebenenfalls durch $C_1$-$C_5$ Alkyl, $C_1$-$C_5$ Alkoxy, Hydroxy oder Halogen substituiert sein kann, einen $C_5$-$C_7$ Cycloalkyl- oder den Phenylrest,
$R_3$ = Wasserstoff, niederes geradkettiges oder verzweigtes $C_1$-$C_5$ Alkyl, das gegebenenfalls durch Hydroxy, $C_1$-$C_5$ Alkylthio, Mercapto oder Phenyl substituiert sein kann, oder gegebenenfalls durch Hydroxy oder $C_1$-$C_5$ Alkoxy substituiertes Phenyl,
$R_4$, $R_6$, $R_6$ und $R_9$ jeweils unabhängig voneinander Wasserstoff oder $C_1$-$C_5$ Alkyl,
$R_5$ und $R_7$ jeweils unabhängig voneinander Wasserstoff, $C_1$ - $C_5$ Alkyl oder gegebenenfalls durch Hydroxy oder $C_1$ - $C_5$ Alkoxy substituiertes Phenyl,
X = Wasserstoff, OH oder die Gruppe -$NR_{10}$ $R_{11}$, wobei $R_{10}$ und $R_{11}$ jeweils unabhängig voneinander Wasserstoff oder $C_1$-$C_5$ Alkyl sein soll,
m bzw. n = 0 oder 1
bedeuten, wobei
$R_3$ und $R_4$ zusammen mit dem Kohlenstoffatom, an das sie gebunden sind, einen Fünf- oder Sechsring,
$R_4$ und $R_6$ zusammen mit den Kohlenstoffatomen, an die sie gebunden sind, einen Fünf- oder Sechsring,
$R_5$ und $R_6$ zusammen mit dem Kohlenstoff, an das sie gebunden sind, einen Fünf- oder Sechsring,
$R_7$ und $R_8$ zusammen mit dem Kohlenstoffatom, an das sie gebunden sind, einen Fünf- oder Sechsring bilden können,
sowie deren pharmakologisch unbedenklichen Salze und optischen Isomere,
dadurch gekennzeichnet, daß man in an sich bekannter Weise

I. eine Verbindung der allgemeinen Formel II

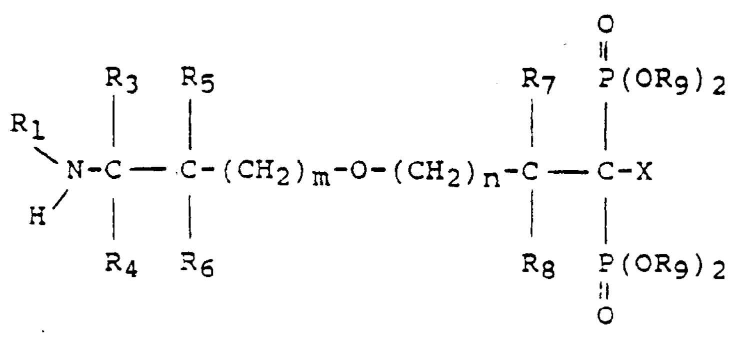

(II),

in der $R_1$, $R_3$ - $R_9$, X, m und n die oben angegebenen Bedeutungen haben, mono- oder dialkyliert und gegebenenfalls die entstandenen Tetraester zu Diestern oder Säuren der allgemeinen Formel I verseift, oder

II. für den Fall, daß X in der allgemeinen Formel I OH bedeutet,

a) eine Carbonsäure der allgemeinen Formel III

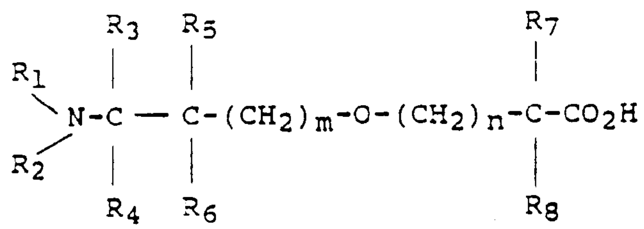

(III),

in der $R_1$ - $R_8$, m und n die oben angegebenen Bedeutungen haben, mit einem Gemisch aus phosphoriger Säure oder Phosphorsäure und einem Phosphorhalogenid bzw. Phosphoroxyhalogenid umsetzt und anschließend zur freien Diphosphonsäure verseift,
oder
b) ein Carbonsäurechlorid der allgemeinen Formel IV

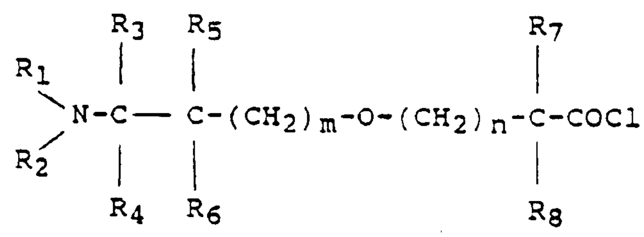

(IV),

in der $R_1$ - $R_8$, m und n die oben genannten Bedeutungen haben, wobei $R_1$ auch eine Acylgruppe oder mit $R_2$ zusammen auch als Schutzgruppe den Phthaloylrest darstellen kann, mit einem Trialkylphosphit der allgemeinen Formel V

$$P(OR')_3, \quad (V),$$

in der R′ für Alkylreste mit 1-4 Kohlenstoffatomen, vorzugsweise Methyl, Ethyl, Isopropyl und Isobutyl steht, zu einem Acylphosphonat der allgemeinen Formel VI

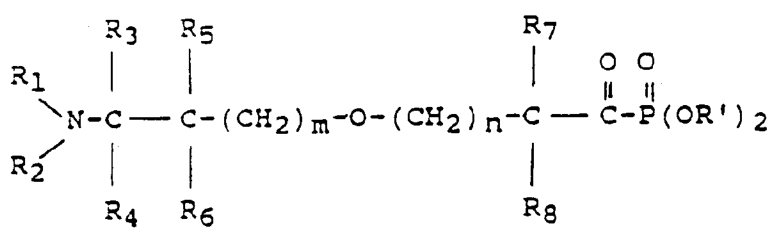

(VI),

in der $R_1$ - $R_8$, m, n und R′ die oben genannten Bedeutungen haben, $R_1$ auch eine Acylgruppe oder mit $R_2$ zusammen auch den Phthaloylrest darstellen kann, umsetzt, anschließend mit einen Dialkylphosphit der allgemeinen Formel VII

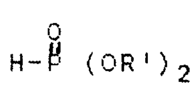

(VII),

in der R′ die oben angegebene Bedeutung hat, zu einem Diphosphonat der allgemeinen Formel VIII

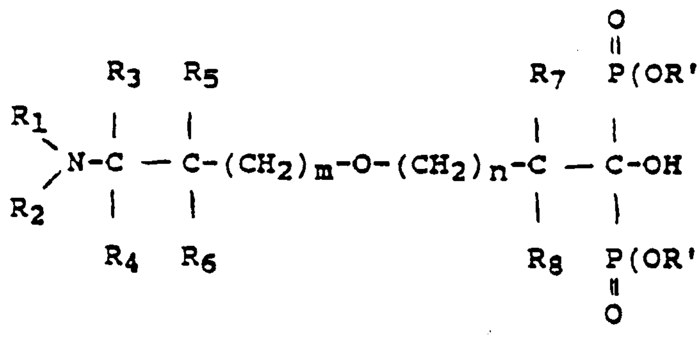 (VIII),

in der $R_1$ - $R_8$, m, n und R′ die oben angegebenen Bedeutungen haben, $R_1$ auch eine Acylgruppe oder mit $R_2$ zusammen auch den Phthaloylrest darstellen kann, reagieren läßt und gegebenenfalls die Phthaloylgruppe durch Hydrazinolyse entfernt und die entstandenen Tetraester zu Diestern oder Säuren der allgemeinen Formel I verseift, wobei unter diesen Bedingungen die als Schutzgruppe verwendete Acyl- bzw. Phthaloylgruppe gleichzeitig abgespalten wird,
oder
c) für den Fall, daß n = O bedeutet, eine Verbindung der allgemeinen Formel IX

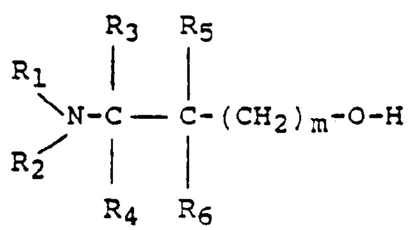 (IX),

in der $R_1$ - $R_8$ und m die oben angegebenen Bedeutungen haben mit einem Epoxid der allgemeinen Formel X

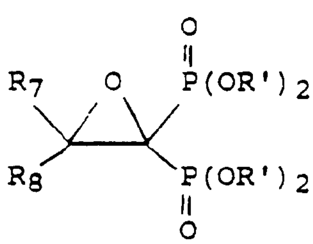 (X),

in der $R_7$, $R_8$ und R′ die oben angegebenen Bedeutungen haben, reagieren läßt und das entstandene Diphosphonsäurederivat der allgemeinen Formel XI

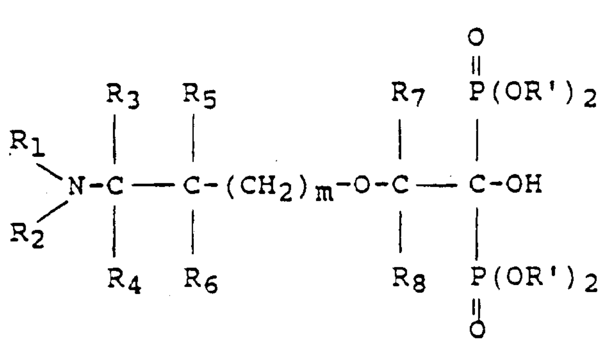 (XI),

gewünschtenfalls zu Diestern oder Säuren verseift,
oder

III. für den Fall, daß X in der allgemeinen Formel I die Gruppe $-NR_{10}R_{11}$ bedeutet, ein Carbonsäurederivat der allgemeinen Formel XII

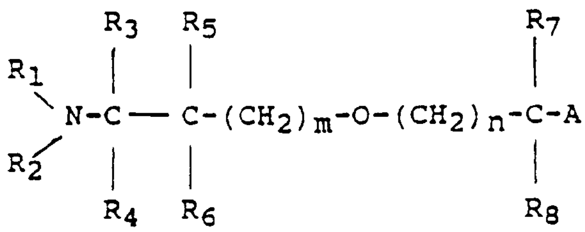

(XII),

in der $R_1$ - $R_8$, m und n die oben angegebenen Bedeutungen haben und A eine Nitril-, Iminoether- oder eine $-CONR_{10}R_{11}$-Gruppe, wobei $R_{10}$ und $R_{11}$ die oben angegebenen Bedeutungen haben, darstellt, mit einer Phosphorverbindung der allgemeinen Formel XIII

$PT_3$ (XIII),

in der T = Halogen, OH oder OR′ bedeutet, wobei R′ die oben angegebene Bedeutung hat, umsetzt und gegebenenfalls anschließend verseift,

oder

IV. für den Fall, daß X in der allgemeinen Formel I Wasserstoff bedeutet,

a) eine Verbindung der allgemeinen Formel XIV

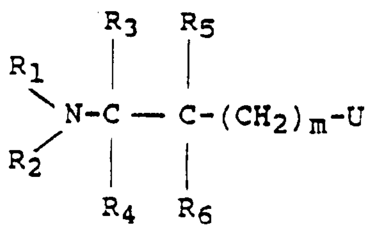

(XIV),

in der $R_1$ - $R_6$ und m die oben angegebenen Bedeutungen haben, wobei $R_1$ auch ein Acyl- oder mit $R_2$ zusammen der Phthaloylrest sein kann, und U eine reaktive Gruppe wie z.B. Halogen oder ein Sulfonat darstellt, mit einem Diphosphonsäurederivat der allgemeinen Formel XV

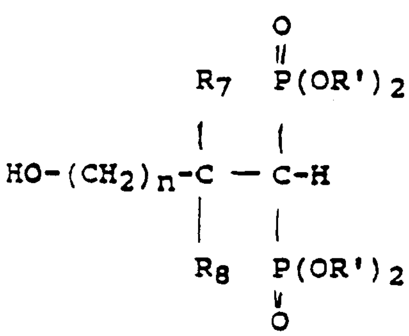

(XV),

in der $R_7$, $R_6$, R′ und n die oben angegebenen Bedeutungen haben, reagieren läßt, und die Phthaloylgruppe gewünschtenfalls durch Hydrazinolyse entfernt und die gebildeten Tetraester gegebenenfalls zu Diestern oder Säuren verseift, wobei unter diesen Bedingungen die als Schutzgruppe verwendete Acyl- oder Phthaloylgruppe gleichzeitig abgespalten wird,

oder

eine Verbindung der allgemeinen Formel IX

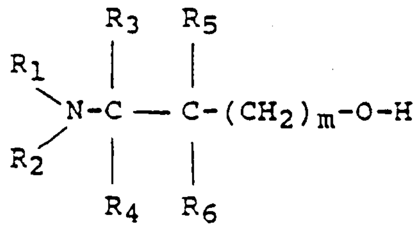

(IX),

in der $R_1$ - $R_6$ und m die oben angegebenen Bedeutungen haben, an eine Verbindung der allgemeinen Formel XVI

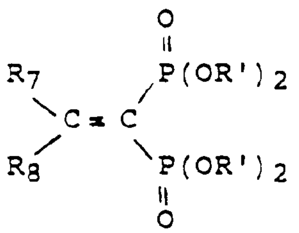

(XVI),

in der $R_7$, $R_8$ und R' die oben angegebenen Bedeutungen haben, addiert und die entstehenden Tetraester gegebenenfalls zu Diestern oder Säuren verseift,
oder
c) eine Verbindung der allgemeinen Formel XVII

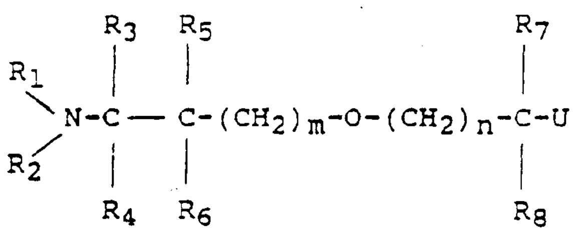

(XVII),

in der $R_1$ - $R_6$, U, m und n die oben angegebenen Bedeutungen haben, wobei $R_1$ auch ein Acyl- oder mit $R_2$ zusammen der Phthaloylrest sein kann, mit einem Diphosphonsäurederivat der allgemeinen Formel XVIII

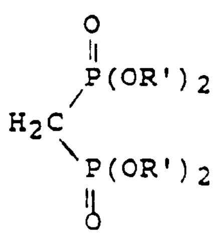

(XVIII),

in der R' die oben angegebene Bedeutung hat, umsetzt, die Phthaloylgruppe gewünschtenfalls durch Hydrazinolyse entfernt und die entstandenen Tetraester gegebenenfalls zu Diestern oder Säuren verseift, wobei unter diesen Bedingungen die als Schutzgruppe verwendete Acyl- oder Phthaloylgruppe gleichzeitig abgespalten wird,
oder
für den Fall, daß $R_6$ Wasserstoff bedeutet,
d) eine Verbindung der allgemeinen Formel XIX

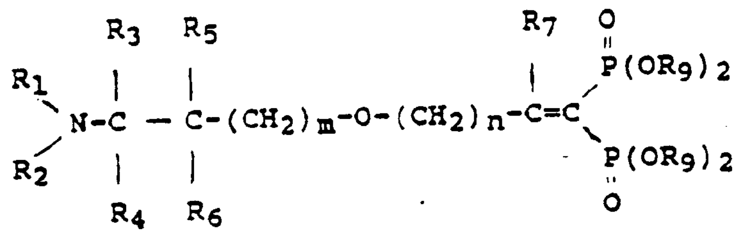 (XIX),

in der $R_1$ - $R_7$, $R_9$, m und n die oben angegebenen Bedeutungen haben, wobei $R_1$ auch eine Acylgruppe darstellen kann, katalytisch hydriert
und anschließend gegebenenfalls die entstandenen Tetraester zu Diestern oder Seifen verseift, wobei dabei auch eine evtl. vorhandene Acylgruppe mit abgespalten werden kann, und die freien Säuren in pharmakologisch unbedenkliche Salze überführt.

4. Verfahren zur Herstellung von Verbindungen gemäß Anspruch 3, in denen $R_1$ Wasserstoff oder Methyl, $R_2$ Wasserstoff oder Methyl, $R_3$ Wasserstoff oder $C_1$-$C_5$ Alkyl, $R_4$ Wasserstoff oder Methyl, $R_5$ Wasserstoff oder Methyl, $R_6$ Wasserstoff, $R_7$ Wasserstoff, $R_5$ Wasserstoff, $R_9$ Wasserstoff, m die Zahl 0 oder 1, n die Zahl 0 und X eine Hydroxylgruppe bedeuten, wobei $R_4$ und $R_5$ zusammen mit den C-Atomen, an die sie gebunden sind, einen Cyclohexyl-Ring und $R_5$ und $R_5$ zusammen mit dem C-Atom, an das sie gebunden sind, einen Spirocyclo-pentan-Ring darstellen.

5. Verbindung nach Anspruch 1 oder 2 ausgewählt aus der Gruppe R,S-5-Amino-3-oxa-hexan-1-hydroxy-1,1-diphosphonsäure, R-5-Amino-3-oxa-hexan-1-hydroxy-1,1-diphosphonsäure und S-5-Amino-3-oxa-hexan-1-hydroxy-1,1-diphosphonsäure.

6. Arzneimittel, enthaltend eine Verbindung gemäß einem der Ansprüche 1, 2 und 5 neben üblichen Träger- und Hilfsstoffen.

7. Verwendung von Verbindungen gemäß einem der Ansprüche 1, 2 und 5 zur Behandlung von Calciumstoffwechselstörungen.

**Claims**

1. Compounds of the general formula I

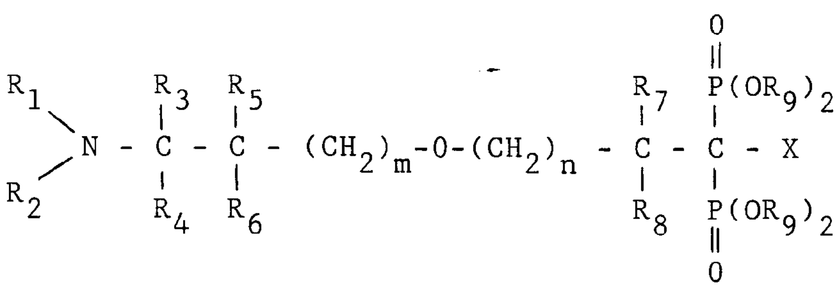 (I)

in which $R_1$ and $R_2$, in each case independently of one another, signifies hydrogen, a straight-chained or branched, saturated or unsaturated alkyl chain with 1 - 9 C-atoms which can possibly be substituted by hydroxyl, $C_1$-$C_5$-alkoxy or $C_1$-$C_5$-alkylthio, a phenyl or a $C_5$-$C_7$-cycloalkyl ring, whereby the phenyl ring can possibly be substituted by $C_1$-$C_5$-alkyl, $C_1$-$C_5$-alkoxy, hydroxyl or halogen, a $C_5$-$C_7$-cycloalkyl or the phenyl radical, $R_3$ = hydrogen, lower straight-chained or branched $C_1$-$C_5$-alkyl, which can possibly be substituted by hydroxyl, $C_1$-$C_5$-alkoxy, $C_1$-$C_5$-alkylthio, mercapto or phenyl, or phenyl possibly substituted by hydroxyl or $C_1$-$C_5$-alkoxy, $R_4$, $R_5$, $R_8$ and $R_9$, in each case independently of one another, signify hydrogen or $C_1$-$C_5$-alkyl, $R_5$ and $R_7$, in each case independently of one another, signify hydrogen, $C_1$-$C_5$-alkyl or phenyl radicals possibly substituted by hydroxyl or $C_1$-$C_5$-alkoxy, X = hydrogen, OH or the group $-NR_{10}R_{11}$, whereby $R_{10}$ and $R_{11}$, in each case independently of one another, are to be hydrogen or $C_1$-$C_5$-alkyl, <u>m</u> and <u>n</u>, respectively = 0 or 1, whereby $R_3$ and $R_4$, together with the carbon atom to which they are attached, can

form a five- or six-membered ring, $R_4$ and $R_6$, together with the carbon atoms to which they are attached, can form a five- or six-membered ring, $R_5$ and $R_6$, together with the carbon atom to which they are attached, can form a five- or six-membered ring, $R_7$ and $R_6$, together with the carbon atom to which they are attached, can form a five- or six-membered ring; as well as their pharmacologically acceptable salts and optical isomers.

2. Compounds of the formula I according to claim 1, in which $R_1$ signifies hydrogen or methyl, $R_2$ hydrogen or methyl, $R_3$ hydrogen or $C_1$-$C_5$-alkyl, $R_4$ hydrogen or methyl, $R_6$ hydrogen or methyl, $R_6$ hydrogen, $R_7$ hydrogen, $R_6$ hydrogen, $R_9$ hydrogen, m the number 0 or 1, n the number 0 and X a hydroxyl group, whereby $R_4$ and $R_6$ together with the C-atoms to which they are attached, represent a cyclohexyl ring and $R_6$ and $R_6$, together with the C-atom to which they are attached, represent a spirocyclopentane ring.

3. Process for the preparation of compounds of the general formula I

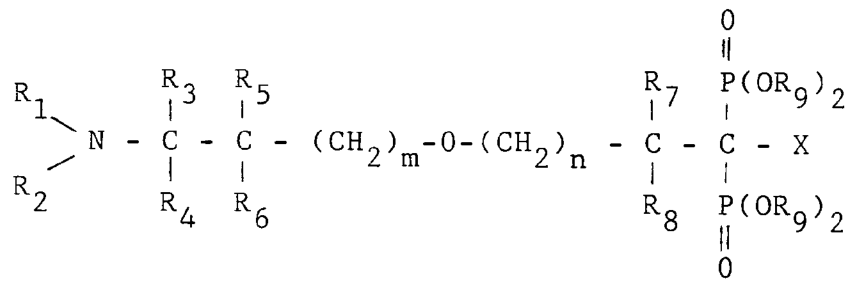

(I)

in which $R_1$ and $R_2$, in each case independently of one another, signifies hydrogen, a straight-chained or branched, saturated or unsaturated alkyl chain with 1 - 9 C-atoms which can possibly be substituted by hydroxyl, $C_1$-$C_5$-alkoxy or $C_1$-$C_5$-alkylthio, a phenyl or a $C_5$-$C_7$-cycloalkyl ring, whereby the phenyl ring can possibly be substituted by $C_1$-$C_5$-alkyl, $C_1$- $C_5$-alkoxy, hydroxyl or halogen, a $C_5$-$C_7$-cycloalkyl or the phenyl radical, $R_3$ = hydrogen, lower straight-chained or branched $C_1$-$C_5$-alkyl, which can possibly be substituted by hydroxyl, $C_1$-$C_5$-alkoxy, $C_1$-$C_5$-alkylthio, mercapto or phenyl, or phenyl possibly substituted by hydroxyl or $C_1$-$C_5$-alkoxy, $R_4$, $R_6$, $R_8$ and $R_9$, in each case independently of one another, signify hydrogen or $C_1$-$C_5$-alkyl, $R_6$ and $R_7$, in each case independently of one another, signify hydrogen, $C_1$-$C_5$-alkyl or phenyl possibly substituted by hydroxyl or $C_1$-$C_5$-alkoxy, X = hydrogen, OH or the group $-NR_{10}R_{11}$, whereby $R_{10}$ and $R_{11}$, in each case independently of one another, are to be hydrogen or $C_1$-$C_5$-alkyl, m and n respectively = 0 or 1, whereby $R_3$ and $R_4$, together with the carbon atom to which they are attached, can form a five- or six-membered ring, $R_4$ and $R_6$, together with the carbon atoms to which they are attached, can form a five- or six-membered ring, $R_5$ and $R_6$, together with the carbon atom to which they are attached, can form a five- or six-membered ring, $R_7$ and $R_6$, together with the carbon atom to which they are attached, can form a five- or six-membered ring; as well as of their pharmacologically acceptable salts and the optical isomers, characterised in that, in per se known manner, one

I. mono- or dialkylates a compound of the general formula II

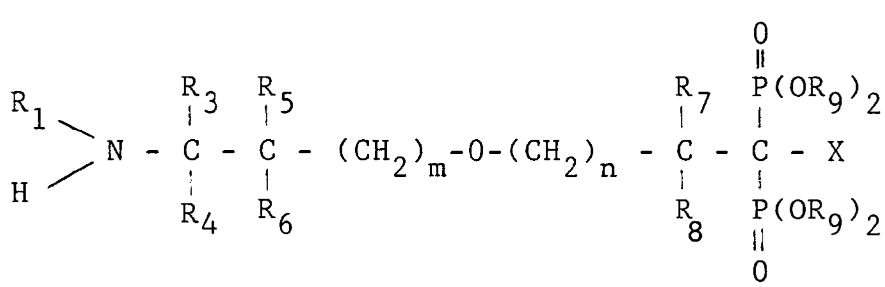

(II)

in which $R_1$, $R_3$ - $R_9$, X, m and n have the above-given meanings, and possibly saponifies the resultant tetraesters to diesters or acids of the general formula I; or

II. for the case that X in general formula I signifies OH,

a) reacts a carboxylic acid of the general formula III

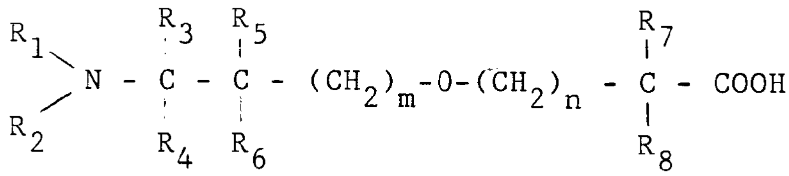

(III)

in which $R_1$ - $R_8$, m and n have the above-given meanings, with a mixture of phosphorous acid or phosphoric acid and a phosphorus halide or phosphorus oxyhalide and subsequently saponifies to the free diphosphonic acid; or

b) reacts a carboxylic acid chloride of the general formula IV

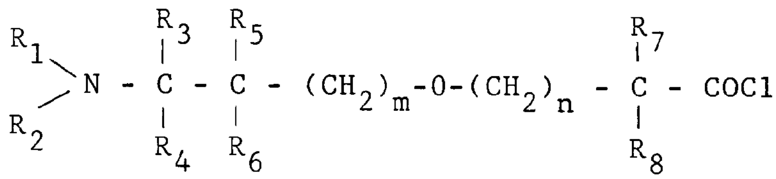

(IV)

in which $R_1$ - $R_8$, m and n have the above-given meanings, whereby $R_1$ can also represent an acyl group or, together with $R_2$, can also represent the phthaloyl radical as protective group, with a trialkyl phosphite of the general formula V

$P(OR')_3$ (V)

in which R′ stands for an alkyl radical with 1 - 4 carbon atoms, preferably methyl, ethyl, isopropyl and isobutyl, to give an acyl phosphonate of the general formula VI

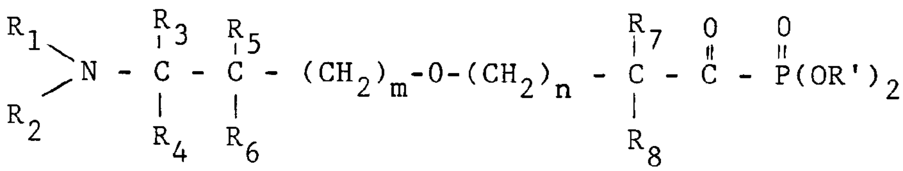

(VI)

in which $R_1$ - $R_8$, M, n and R′ have the above-given meanings, $R_1$ can also represent an acyl group or, together with $R_2$, can also represent the phthaloyl radical, subsequently allows to react with a dialkyl phosphite of the general formula VII

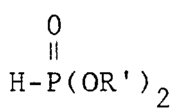

(VII),

in which R′ has the above-given meaning, to give a diphosphonate of the general formula VIII

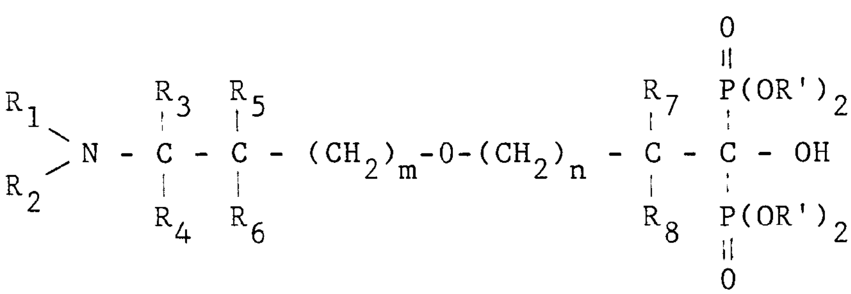

(VIII)

in which $R_1$ - $R_8$, m, n and R′ have the above-given meanings, and $R_1$ can also represent an acyl radical or, together with $R_2$, can also represent the phthaloyl radical, and possibly removes the phthaloyl group by hydrazinolysis and saponifies the resultant tetraesters to diesters or acids of the general formula I, whereby, under these conditions, the acyl or phthaloyl group used as protective group is simultaneously split off; or

c) for the case that n = 0, a compound of the general formula IX

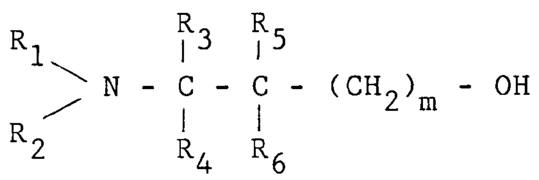

(IX)

in which $R_1$ - $R_6$ and m have the above-given meanings, is allowed to react with an epoxide of the general formula X

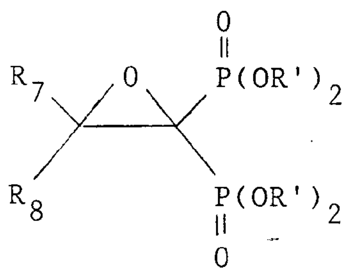

(X)

in which $R_7$, $R_8$ and R′ have the above-given meanings, and, if desired, saponifies the resultant diphosphonic acid derivative of the general formula XI

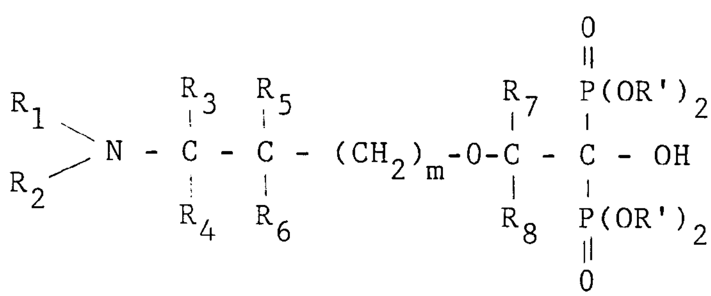

(XI)

to diesters or acids; or

III. for the case that X in general formula I signifies the group $-NR_{10}R_{11}$, reacts a carboxylic acid derivative of the general formula XII

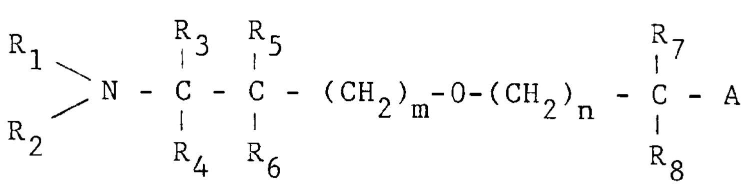

(XII)

in which $R_1$ - $R_8$, m and n have the above-given meanings and A represents a nitrile, imino ether or a $-CONR_{10}R_{11}$ group, whereby $R_{10}$ and $R_{11}$ have the above-given meanings, with a phosphorus compound of the general formula XIII

$$PT_3 \qquad (XIII)$$

in which T = halogen, OH or OR′, whereby R′ has the above-given meaning, and possibly subsequently saponifies; or

IV. for the case that X in the general formula I signifies hydrogen,

a) allows a compound of the general formula XIV

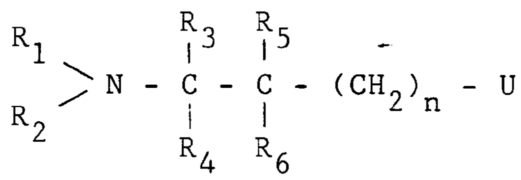

(XIV)

in which $R_1$ - $R_6$ and m have the above-given meanings, whereby $R_1$ can also be an acyl or, together with $R_2$, the phthaloyl radical and U represents a reactive group, such as e.g. halogen or a sulphonate, to react with a diphosphonic acid derivative of the general formula XV

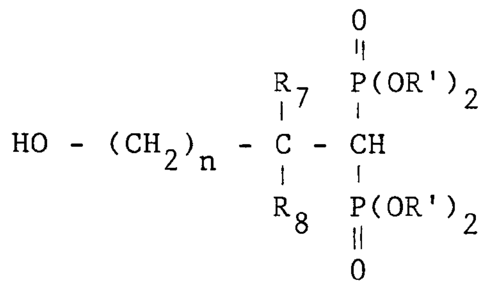

(XV)

in which $R_7$, $R_8$, R′ and n have the above-given meanings, and, if desired, removes the phthaloyl group by hydrazinolysis and saponifies the tetraesters formed to diesters or acids whereby, under these conditions, the acyl or phthaloyl group used as protective group, is split off simultaneously; or

b) adds a compound of the general formula IX

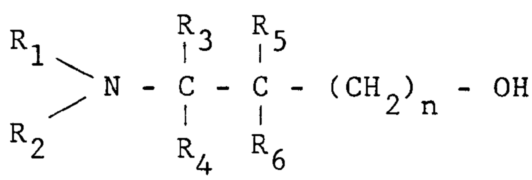

(IX)

in which $R_1$ - $R_6$ and m have the above-given meanings, to a compound of the general formula XVI)

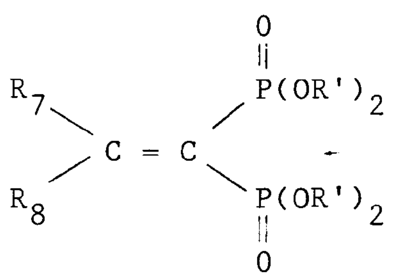

(XVI)

in which $R_7$, $R_8$ and R′ have the above-given meanings, and possibly saponifies the resultant tetraesters to diesters or acids; or

c) reacts a compound of the general formula XVII

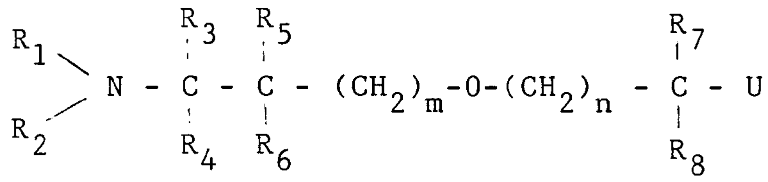 (XVII)

in which $R_1$ - $R_8$, U, m and n have the above-given meanings, whereby $R_1$ can also be an acyl or, together with $R_2$, a phthaloyl radical, with a diphosphonic acid derivative of the general formula XVIII

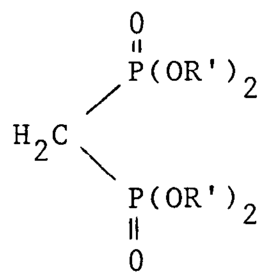 (XVIII)

in which R′ has the above-given meaning, if desired removes the phthaloyl group by hydrazinolysis and possibly saponifies the resultant tetraesters to diesters or acids, whereby, under these conditions, the acyl or phthaloyl group used as protective group is simultaneously split off; or, for the case that $R_8$ signifies hydrogen,

d) catalytically hydrogenates a compound of the general formula XIX

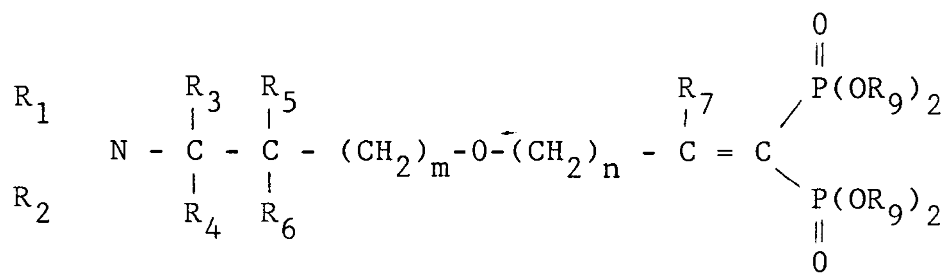 (XIX)

in which $R_1$ - $R_7$, $R_9$, m and n have the above-given meanings, whereby $R_1$ can also represent an acyl group, and subsequently, if desired, saponifies the resultant tetraesters to diesters or acids, whereby an acyl group possibly present can thereby also be split off at the same time and the free acids converted into pharmacologically acceptable salts.

4. Process for the preparation of compounds according to claim 3, in which $R_1$ signifies hydrogen or methyl, $R_2$ hydrogen or methyl, $R_3$ hydrogen or $C_1$-$C_5$-alkyl, $R_4$ hydrogen or methyl, $R_5$ hydrogen or methyl, $R_8$ hydrogen, $R_7$ hydrogen, $R_8$ hydrogen, $R_9$ hydrogen, m the number 0 or 1, n the number 0 and X a hydroxyl group, whereby $R_4$ and $R_8$, together with the C-atoms to which they are attached, represent a cyclohexyl ring and $R_8$ and $R_8$, together with the C-atom to which they are attached, represent a spirocyclopentane ring.

5. Compounds according to claim 1 or 2 selected from the group R,S-5-amino-3-oxahexane-1-hydroxy-1,1-diphosphonic acid, R-5-amino-3-oxahexane-1-hydroxy-1,1-diphosphonic acid and S-5-amino-3-oxahexane-1-hydroxy-1,1-diphosphonic acid.

6. Medicaments containing a compound according to one of claims 1, 2 and 5, besides usual carrier and adjuvant materials.

7. Use of compounds according to one of claims 1, 2 and 5 for the treatment of calcium metabolism disturbances.

## Revendications

1. Composés de formule générale I

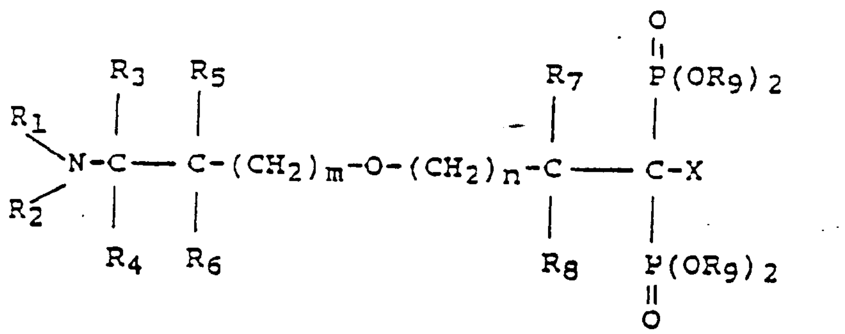

(I)

dans laquelle

$R_1$ et $R_2$ représentent dans chaque cas indépendamment l'un de l'autre un atome d'hydrogène, un groupe alkyle à chaîne droite ou ramifiée, saturé ou insaturé, comportant 1-9 atomes de carbone, qui peut être substitué éventuellement par un groupe hydroxy, alcoxy en $C_1$-$C_5$ ou alkylthio en $C_1$-$C_5$, un noyau phényle ou un noyau cycloalkyle en $C_5$-$C_7$, le noyau phényle pouvant être substitué éventuellement par un groupe alkyle en $C_1$-$C_5$, alcoxy en $C_1$-$C_5$, hydroxy ou halogène, un radical cycloalkyle en $C_5$-$C_7$ ou le radical phényle,

$R_3$ représente un atome d'hydrogène, un groupe alkyle inférieur, en $C_1$-$C_5$, à chaîne droite ou ramifiée, qui peut être éventuellement substitué par un groupe hydroxy, alcoxy en $C_1$-$C_5$, alkylthio en $C_1$-$C_5$, mercapto ou phényle, ou éventuellement un groupe phényle substitué par un groupe hydroxy ou un groupe alcoxy en $C_1$-$C_5$,

$R_4$, $R_6$, $R_8$ et $R_9$ représentent dans chaque cas indépendamment l'un de l'autre un atome d'hydrogène ou un groupe alkyle en $C_1$-$C_5$,

$R_5$ et $R_7$ représentent dans chaque cas indépendamment l'un de l'autre un atome d'hydrogène ou un groupe alkyle en $C_1$-$C_5$, ou éventuellement un groupe phényle substitué par un groupe hydroxy ou un groupe alcoxy en $C_1$-$C_5$,

X représente un atome d'hydrogène, un groupe OH ou le groupe $-NR_{10}R_{11}$, où $R_{10}$ et $R_{11}$ doivent représenter dans chaque cas indépendamment l'un de l'autre un atome d'hydrogène ou un groupe alkyle en $C_1$-$C_5$,

m et n valent 0 ou 1

$R_3$ et $R_4$ pouvant former, conjointement avec l'atome de carbone auquel ils sont liés, un cycle à cinq ou six chaînons,

$R_4$ et $R_6$ pouvant former, conjointement avec l'atome de carbone auquel ils sont liés, un cycle à cinq ou six chaînons,

$R_5$ et $R_6$ pouvant former, conjointement avec l'atome de carbone auquel ils sont liés, un cycle à cinq ou six chaînons,

$R_7$ et $R_6$ pouvant former, conjointement avec l'atome de carbone auquel ils sont liés, un cycle à cinq ou six chaînons,

ainsi que leurs sels pharmacologiquement acceptables et leurs isomères optiques.

2. Composés de formule I selon la revendication 1, dans laquelle $R_1$ représente un atome d'hydrogène ou un groupe méthyle, $R_2$ représente un atome d'hydrogène ou un groupe méthyle, $R_3$ représente un atome d'hydrogène ou un groupe alkyle en $C_1$-$C_5$, $R_4$ représente un atome d'hydrogène ou un groupe méthyle, $R_5$ représente un atome d'hydrogène ou un groupe méthyle, $R_6$ représente un atome d'hydrogène, $R_7$ représente un atome d'hydrogène, $R_8$ représente un atome d'hydrogène, $R_9$ représente un atome d'hydrogène, m vaut 0 ou 1, n vaut 0 et X représente un groupe hydroxyle, $R_4$ et $R_6$ pouvant former, conjointement avec l'atome de carbone auquel ils sont liés, un noyau cyclohexyle et $R_6$ et $R_6$ pouvant former, conjointement avec l'atome de carbone auquel ils sont liés, un noyau spirocyclopentane.

3. Procédé de préparation de composés de formule générale I

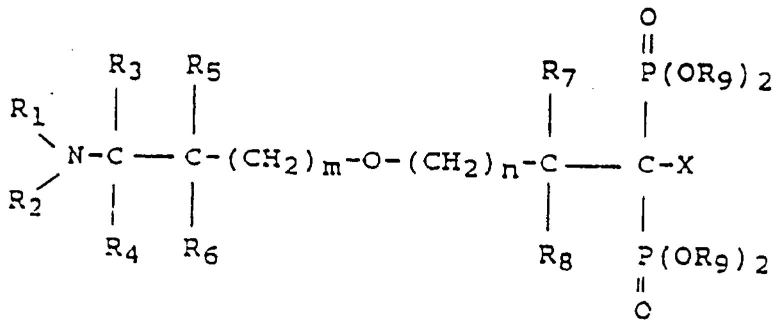

(I),

dans laquelle

$R_1$ et $R_2$ représentent dans chaque cas indépendamment l'un de l'autre un atome d'hydrogène, un groupe alkyle à chaîne droite ou ramifiée, saturé ou insaturé, comportant 1-9 atomes de carbone, qui peut être substitué éventuellement par un groupe hydroxy, alcoxy en $C_1$-$C_5$ ou alkylthio en $C_1$-$C_5$, un noyau phényle ou un noyau cycloalkyle en $C_5$-$C_7$, le noyau phényle pouvant être substitué éventuellement par un groupe alkyle en $C_1$-$C_5$, alcoxy en $C_1$-$C_5$ hydroxy ou halogène, un radical cycloalkyle en $C_5$-$C_7$ ou le radical phényle,

$R_3$ représente un atome d'hydrogène, un groupe alkyle inférieur, en $C_1$-$C_5$, à chaîne droite ou ramifiée, qui peut être éventuellement substitué par un groupe hydroxy, alcoxy en $C_1$-$C_5$, alkylthio en $C_1$-$C_5$, mercapto ou phényle, ou éventuellement un groupe phényle substitué par un groupe hydroxy ou un groupe alcoxy en $C_1$-$C_5$,

$R_4$, $R_6$, $R_8$ et $R_9$ représentent dans chaque cas indépendamment l'un de l'autre un atome d'hydrogène ou un groupe alkyle en $C_1$-$C_5$,

$R_5$ et $R_7$ représentent dans chaque cas indépendamment l'un de l'autre un atome d'hydrogène ou un groupe alkyle en $C_1$-$C_5$, ou éventuellement un groupe phényle substitué par un groupe hydroxy ou un groupe alcoxy en $C_1$-$C_5$,

X représente un atome d'hydrogène, un groupe OH ou le groupe $-NR_{10}R_{11}$, où $R_{10}$ et $R_{11}$ doivent représenter dans chaque cas indépendamment l'un de l'autre un atome d'hydrogène ou un groupe alkyle en $C_1$-$C_5$,

m et n valent 0 ou 1

$R_3$ et $R_4$ pouvant former, conjointement avec l'atome de carbone auquel ils sont liés, un cycle à cinq ou six chaînons,

$R_4$ et $R_6$ pouvant former, conjointement avec l'atome de carbone auquel ils sont liés, un cycle à cinq ou six chaînons,

$R_5$ et $R_6$ pouvant former, conjointement avec l'atome de carbone auquel ils sont liés, un cycle à cinq ou six chaînons,

$R_7$ et $R_6$ pouvant former, conjointement avec l'atome de carbone auquel ils sont liés, un cycle à cinq ou six chaînons,

ainsi que leurs sels pharmacologiquement acceptables et leurs isomères optiques,

caractérisé en ce que, de manière connue en soi,

I. on soumet un composé de formule générale II

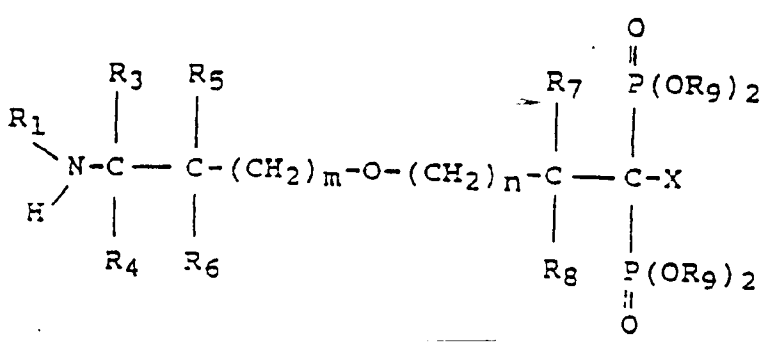

(II),

dans laquelle $R_1$, $R_3$-$R_9$, X, m et n ont les significations mentionnées ci-dessus, à une mono- ou dialkylation et le cas échéant, on transforme le tétraester obtenu, par saponification, en un diester ou un acide

de formule générale I,

ou

II. dans le cas où X, dans la formule générale I, représente un groupe OH,

a) on fait réagir un acide carboxylique de formule générale III

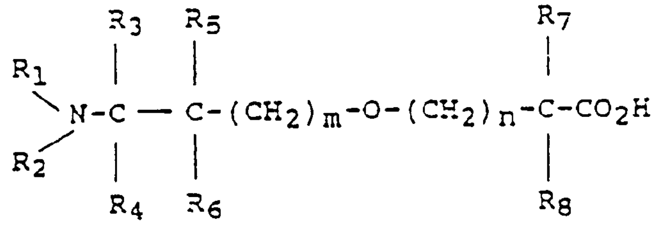 (III),

dans laquelle $R_1$-$R_8$, m et n ont les significations mentionnées ci-dessus, avec un mélange d'acide phosphoreux ou d'acide phosphorique et d'un halogénure phosphorique ou d'un halogénure oxyphosphorique, et ensuite, on saponifie en acide diphosphonique libre,

ou

b) on fait réagir un chlorure d'acide carboxylique de formule générale IV

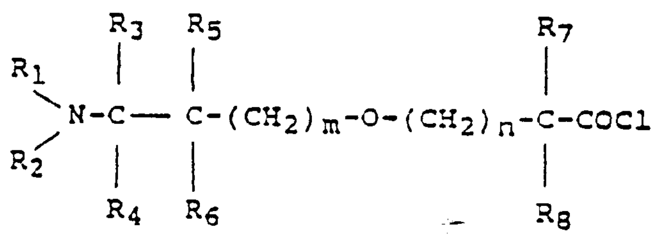 (IV),

dans laquelle $R_1$-$R_8$, m et n ont les significations mentionnées ci-dessus, $R_1$ pouvant également représenter un groupe acyle, ou conjointement avec $R_2$, en tant que groupe de protection, le radical phtaloyle, avec un trialkylphosphite de formule générale V

$$P(OR')_3 \qquad (V),$$

dans laquelle R′ représente un groupe alkyle comportant 1-4 atomes de carbone, de préférence un groupe méthyle, éthyle, isopropyle et isobutyle, pour obtenir un phosphonate d'acyle de formule générale VI

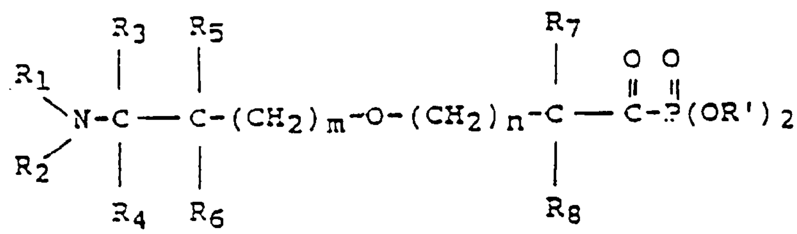

(VI),

dans laquelle $R_1$-$R_8$, m, n et R′ ont les significations mentionnées ci-dessus, $R_1$ pouvant également représenter un groupe acyle, ou conjointement avec $R_2$, le radical phtaloyle, ensuite, on fait réagir avec un dialkylphosphite de formule générale VII

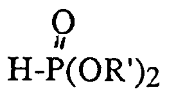 (VII),

dans laquelle R′ a la signification mentionnée ci-dessus, en un diphosphonate de formule générale VIII

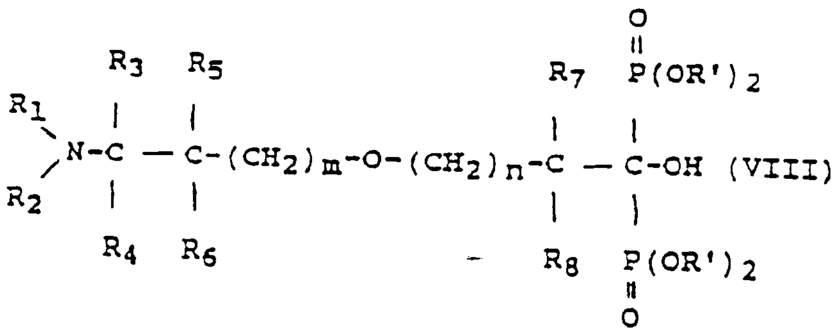

dans laquelle $R_1$-$R_8$, m, n et R′ ont les significations mentionnées ci-dessus, $R_1$ pouvant également représenter un groupe acyle, ou conjointement avec $R_2$, le radical phtaloyle, et éventuellement, on élimine le groupe phtaloyle par hydrazinolyse et on saponifie le tétraester formé en diester ou en acide de formule générale I, le groupe acyle ou phtaloyle, utilisé comme groupe de protection, étant dans ces conditions coupé simultanément,

ou

c) dans le cas où n vaut 0, on fait réagir un composé de formule générale IX

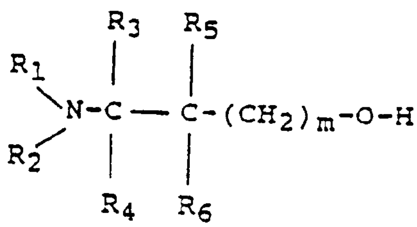

(IX),

dans laquelle $R_1$-$R_8$ et m ont les significations mentionnées ci-dessus, avec un époxyde de formule générale X

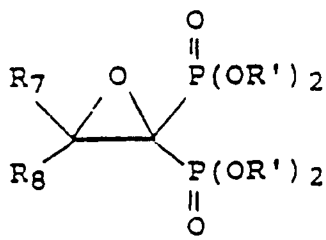

(X),

dans laquelle $R_7$, $R_8$ et R′ ont les significations mentionnées ci-dessus, et le cas échéant, on saponifie le dérivé d'acide diphosphonique obtenu, de formule générale XI

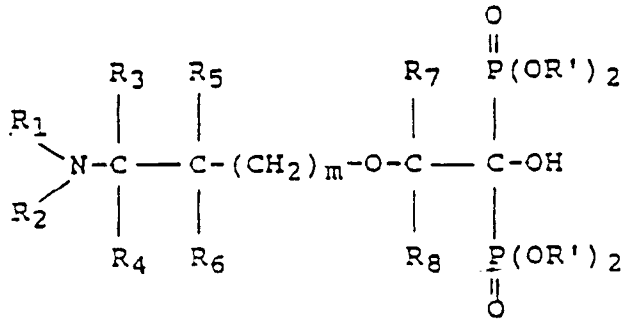

(XI),

en diester ou acide,

ou

III. dans le cas où X, dans la formule générale I, représente le groupe $-NR_{10}R_{11}$, on fait réagir un dérivé d'acide carboxylique de formule générale XII

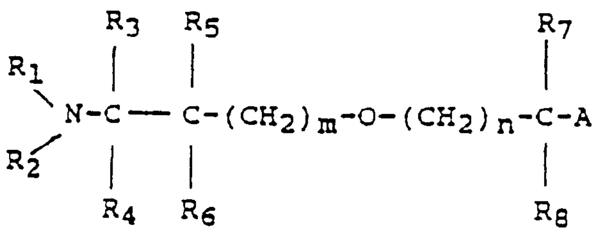

(XII),

dans laquelle $R_1$-$R_8$, m et n ont les significations mentionnées, et A représente un groupe nitrile, iminoéther ou $-CONR_{10}R_{11}$, dans lequel $R_{10}$ et $R_{11}$ ont les significations mentionnées plus haut, avec un composé du phosphore de formule générale XIII

$PT_3$ (XIII),

dans laquelle T représente un groupe halogène, OH ou OR′, R′ ayant la même signification que ci-dessus, et ensuite, on effectue éventuellement une saponification,

ou

IV. dans le cas où X, dans la formule générale I, représente un atome d'hydrogène,

a) on fait réagir un composé de formule XIV

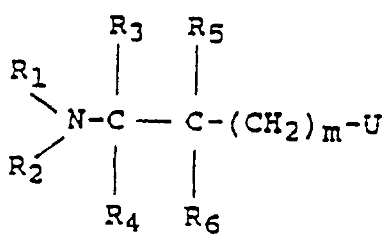

(XIV),

dans laquelle $R_1$-$R_8$ et m ont les significations mentionnées ci-dessus, $R_1$ pouvant également représenter un groupe acyle, ou conjointement avec $R_2$, le radical phtaloyle, et U représente un groupe réactif tel que par exemple un atome d'halogène ou un sulfonate, avec un dérivé d'acide diphosphonique, de formule générale XV

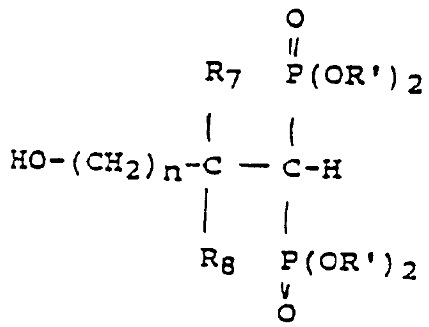

(XV),

dans laquelle $R_7$, $R_8$, R′ et n ont les significations mentionnées ci-dessus, et on élimine éventuellement le groupe phtaloyle par hydrazinolyse, et le cas échéant, on effectue une saponification en diester ou acide, le groupe acyle ou phtaloyle, utilisé comme groupe de protection, étant dans ces conditions coupé simultanément,

ou

b) on effectue l'addition d'un composé de formule générale IX

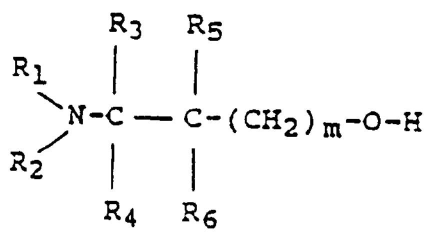

(IX),

dans laquelle $R_1$-$R_6$ et m ont les significations mentionnées ci-dessus, sur un composé de formule générale XVI

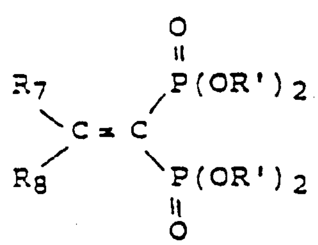

(XVI),

dans laquelle $R_7$, $R_8$ et R′ ont les significations mentionnées ci-dessus, et le cas échéant, on saponifie le tétraester formé en diester ou acide,

ou

c) on fait réagir un composé de formule XVII

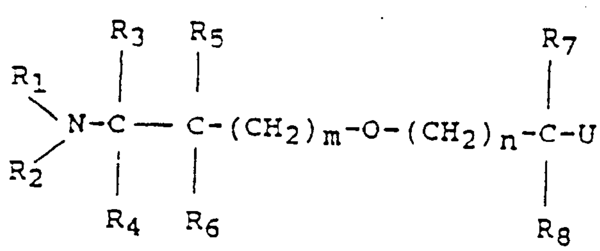

(XVII),

dans laquelle $R_1$-$R_8$, U, m, et n ont les significations mentionnées ci-dessus, $R_1$ pouvant également représenter un groupe acyle, ou conjointement avec $R_2$, le radical phtaloyle, avec un dérivé d'acide diphosphonique, de formule générale XVIII

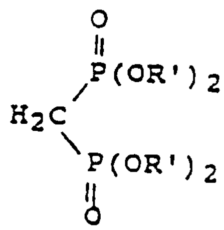

(XVIII),

dans laquelle R′ a la signification mentionnée plus haut, on élimine éventuellement le groupe phtaloyle par hydrazinolyse, et l'on saponifie éventuellement le tétraester formé en diester ou en acide, le groupe acyle ou phtaloyle, utilisé comme groupe de protection, étant dans ces conditions coupé simultanément,

ou

dans le cas où $R_8$ représente un atome d'hydrogène,

d) on soumet un composé de formule générale XIX

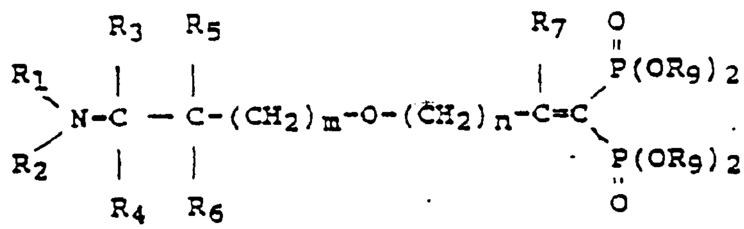

(XIX),

dans laquelle $R_1$-$R_7$, $R_9$, m, et n ont les significations mentionnées ci-dessus, $R_1$ pouvant également représenter un groupe acyle, à une hydrogénation catalytique,

et ensuite, on saponifie éventuellement le tétraester formé en diester ou en acide, un groupe acyle éventuellement présent pouvant être coupé également, et l'on transforme l'acide libre en sel pharmacologiquement acceptable.

4. Procédé pour la préparation de composés selon la revendication 3, où $R_1$ représente un atome d'hydrogène ou un groupe méthyle, $R_2$ représente un atome d'hydrogène ou un groupe méthyle, $R_3$ représente un atome d'hydrogène ou un groupe alkyle en $C_1$-$C_5$, $R_4$ représente un atome d'hydrogène ou un groupe méthyle, $R_5$ représente un atome d'hydrogène ou un groupe méthyle, $R_6$ représente un atome d'hydrogène, $R_7$ représente un atome d'hydrogène, $R_8$ représente un atome d'hydrogène, $R_9$ représente un atome d'hydrogène, m vaut 0 ou 1, n vaut 0 et X représente un groupe hydroxyle, $R_4$ et $R_6$ pouvant former, conjointement avec l'atome de C auquel ils sont liés, un noyau cyclohexyle et $R_6$ et $R_6$ pouvant former, conjointement avec l'atome de C auquel ils sont liés, un noyau spirocyclopentane.

5. Composé selon la revendication 1 ou 2, choisi dans le groupe formé par l'acide R,S -5-amino-3-oxa-hexane-1-hydroxy-1, 1-diphosphonique, l'acide R-5-amino-3-oxa-hexane-1-hydroxy-1, 1-diphosphonique et l'acide S-5-amino-3-oxa-hexane-1-hydroxy-1, 1-diphosphonique.

6. Médicament contenant un composé selon l'une quelconque des revendications 1, 2 et 5, en plus de supports et adjuvants usuels.

7. Utilisation de composés selon l'une quelconque des revendications 1, 2 et 5, pour le traitement des troubles du métabolisme calcique.